# EUROPEAN PATENT SPECIFICATION

(11) **EP 2 976 064 B1**
(45) Date of publication and mention of the grant of the patent: **19.04.2017**
(21) Application number: 14717202.7
(22) Date of filing: 19.03.2014
(51) Int. Cl.: A61K 9/16, A61K 9/20, A61K 9/28, A61K 9/50, A61K 31/436

(54) **PHARMACEUTICAL COMPOSITIONS COMPRISING EVEROLIMUS**
PHARMAZEUTISCHE ZUSAMMENSETZUNGEN MIT EVEROLIMUS
COMPOSITIONS PHARMACEUTIQUES COMPRENANT DE L'ÉVÉROLIMUS

(30) Priority: 19.03.2013 US 201361803300 P
(43) Date of publication of application: 27.01.2016
(73) Proprietor: Novartis AG, 4056 Basel (CH)
(72) Inventor: DIEDERICH, Anke, CH-4002 Basel (CH); KUNZLER, Hans-Ulrich, CH-4002 Basel (CH); GRANER, Oliver, CH-4002 Basel (CH)
(74) Representative: Kristl, Jernej
(86) International application number: PCT/IB2014/059965
(87) International publication number: WO 2014/147567

(56) References cited:
- WO-A1-2013/050419
- WO-A2-2008/016633
- WO-A2-2010/056754

## Description

### Field of the Invention

The present invention relates to two new pharmaceutical formulations comprising 40-O-(2-hydroxy)ethyl-rapamycin. The present invention relates to a new process for the preparation of the two formulations of 40-O-(2-hydroxy)ethyl-rapamycin. The pharmaceutical formulations disclosed herein are particularly useful as a medicament, especially for the treatment of a tumor disease or in the prophylaxis of organ rejection.

### Description of Background Art

40-O-(2-hydroxy)ethyl-rapamycin, is an orally active rapamycin derivative which is described for instance in Example 8 of WO94/09010. 40-O-(2-hydroxy)ethyl-rapamycin has been first approved as immunosuppressant in 2003 and is available to patients now in > 80 countries under the name of Certican© / Zortress©, e.g. for the prevention of organ rejection, or under the name Afinitor© / Votubia© for the treatment of tumor diseases. They are all in the form of immediate release formulations.

40-O-(2-hydroxy)ethyl-rapamycin (everolimus, RAD001) formulations and methods for the preparation of such formulations are disclosed in e.g. WO97/03654 relating to oral pharmaceutical compositions for rapamycins, such as for instance 40-O-(2-hydroxy)ethyl-rapamycin, which are in the form of a solid dispersion. WO03/028705 discloses oral pharmaceutical compositions for rapamycins, such as for instance 40-O-(2-hydroxy)ethyl-rapamycin, comprising colloidal silicon dioxide to promote disintegration. WO05/034916 describes inter alia pharmaceutical compositions for fixed-dose combinations comprising mycophenolic acid (including its salt or a prodrug) and RAD001 which are in multiparticulate form and wherein the combinations of active ingredient particles are preferably enterically coated.

### Summary of the invention

The object of the present disclosure was to provide an improved pharmaceutical formulation comprising 40-O-(2-hydroxy)ethyl-rapamycin. Particularly, the object was to provide 40-O-(2-hydroxy)ethyl-rapamycin in a clinically safe oral solid dosage form due to improved drug product stability and controlled oral bioavailability.

In one aspect the present invention provides a pharmaceutical formulation comprising a first part and a second part, wherein the first part comprises a layer with more than 40 wt % of 40-O-(2-hydroxy)ethyl-rapamycin and the second part releases more than 85 % of 40-O-(2-hydroxy)ethyl-rapamycin of the second part in less than 60 minutes.

In another aspect the present invention provides a pharmaceutical formulation comprising 40-O-(2-hydroxy)ethyl-rapamycin in a first layer and a surfactant in a second layer, wherein the second layer is beneath the first layer, or enclosed by the first layer.

In the third aspect the present invention provides a process for preparing the formulation of the present disclosure.

In yet another aspect the present invention provides a pharmaceutical formulation according to the two aforementioned aspects for use as a medicament.

### Description of the invention, its advantages and preferred embodiments:

The aspects, advantageous features and preferred embodiments of the present invention summarized in the following items, respectively alone or in combination, further contribute to solving the object of the invention:
1. A pharmaceutical formulation comprising a first part and a second part, wherein the first part comprises a layer with more than 40 wt % of 40-O-(2-hydroxy)ethyl-rapamycin and the second part releases more than 85 wt % of 40-O-(2-hydroxy)ethyl-rapamycin of the second part in less than 60 minutes.
2. A pharmaceutical formulation according to item 1, wherein the first part comprises a layer with more than 45 wt %, 50 wt %, 60 wt %, 70 wt %, 80 wt %, or 90 wt % of 40-O-(2-hydroxy)ethyl-rapamycin, preferably more than 60 wt %.
3. A pharmaceutical formulation according to item 1 or 2, wherein the first part comprises a layer with between 50 to 80 wt % of 40-O-(2-hydroxy)ethyl-rapamycin.
4. A pharmaceutical formulation according to any one of items 1 or 3, wherein the first part comprises a layer with between 55 to 70 wt % of 40-O-(2-hydroxy)ethyl-rapamycin.
5. A pharmaceutical formulation according to any one of items 1 or 4, wherein the first part comprises a layer with between 60 to 70 wt % of 40-O-(2-hydroxy)ethyl-rapamycin.
6. A pharmaceutical formulation according to item 1 or 5, wherein the second part releases more than 80 % or 90 % of 40-O-(2-hydroxy)ethyl-rapamycin of the second part in less than 30 minutes, preferably substantially all 40-O-(2-hydroxy)ethyl-rapamycin of the second part in less than 30 minutes.
7. A pharmaceutical formulation according to any one of items 1 to 6, wherein the weight ratio of 40-O-(2-hydroxy)ethyl-rapamycin in the first and the second part is from 2:5 to 20:1, preferably is from 5:1 to 20:1; particularly is from 8:1 to 12:1, specifically is 10:1.
8. A pharmaceutical formulation according to any one of the previous items, wherein the first part and/or the second part is in a form of a minitablet, pellet, microparticle, microcapsule, granule, bead, tablet, a coating layer of a coated minitablet, pellet, microparticle, microcapsule, granule, bead, tablet, or a layer of a double or multilayer tablet.
9. A pharmaceutical formulation according to any one of the previous items, wherein the first part is in a form of a coating and the second part is in a form of a coating.
10. A pharmaceutical formulation according to item 9, wherein the first and the second part are in the form of a coating of a coated bead or pellet.
11. A pharmaceutical formulation according to item 8, wherein the first part is in the form of a pellet or a microcapsule, and the second part is in the form of a minitablet or tablet.
12. A pharmaceutical formulation according to any one of the previous items, wherein the second part comprises a layer with less than 40 wt % of 40-O-(2-hydroxy)ethyl-rapamycin, preferably less than 20 wt% of 40-O-(2-hydroxy)ethyl-rapamycin.
13. A pharmaceutical formulation according to any one of the previous items, wherein the formulation further comprises a surfactant.
14. A pharmaceutical formulation according to item 13, wherein the surfactant is in a coating, wherein the coating with the surfactant is enclosed at least by the layer with more than 40 wt % of 40-O-(2-hydroxy)ethyl-rapamycin.
15. A pharmaceutical formulation comprising 40-O-(2-hydroxy)ethyl-rapamycin in a first layer and a surfactant in a second layer, wherein the second layer is beneath the first layer.
16. A pharmaceutical formulation comprising 40-O-(2-hydroxy)ethyl-rapamycin in a first layer and a surfactant in a second layer according to item 15, wherein the first and the second layer are coatings.
17. A pharmaceutical formulation comprising 40-O-(2-hydroxy)ethyl-rapamycin in a first layer and a surfactant in a second layer according to item 15 or 16, wherein the second layer with the surfactant is enclosed at least by the first layer.
18. A pharmaceutical formulation comprising 40-O-(2-hydroxy)ethyl-rapamycin in a first layer and a surfactant in a second layer according to any one of items 15 to 17, wherein 40-O-(2-hydroxy)ethyl-rapamycin in the first layer is in a solid dispersion and the solid dispersion comprises 18 to 50 wt % of 40-O-(2-hydroxy)ethyl-rapamycin.
19. A pharmaceutical formulation according to any one of the previous items, wherein the formulation comprises a further coating.
20. A pharmaceutical formulation according to item 19, wherein the coating is extended release coating or a protection coating.
21. A pharmaceutical formulation according to item 20, wherein the extended release coating comprises polymer with pH independent water solubility.
22. A pharmaceutical formulation according to item 21, wherein the polymer is cellulose ether, polymethacrylate, polyvinylacetate or a combination thereof.
23. A pharmaceutical formulation according to item 21 or 22, wherein the polymer is ethyl cellulose.
24. A pharmaceutical formulation according to any one of items 10 to 20, wherein the coating further comprises a water soluble polymer.
25. A pharmaceutical formulation according to item 20, wherein the protection coating is encaging the layer comprising 40-O-(2-hydroxy)ethyl-rapamycin or is separating the layer comprising 40-O-(2-hydroxy)ethyl-rapamycin from adjacent layer.
26. A pharmaceutical formulation comprising 40-O-(2-hydroxy)ethyl-rapamycin in a first layer and a surfactant in a second layer according to any one of items 15 to 25, wherein the pharmaceutical formulation is in a form of a pellet.
27. A pharmaceutical formulation comprising 40-O-(2-hydroxy)ethyl-rapamycin in a first layer and a surfactant in a second layer according to 26, wherein the 40-O-(2-hydroxy)ethyl-rapamycin load is between 1.4 to 15 wt%, preferably between 5 and 11 wt %, particularly between 7 and 9 wt %.
28. A pharmaceutical formulation according to item 20 or 27, wherein the protection coating comprises talc and/or hypromellose, preferably hypromellose.
29. A pharmaceutical formulation according to any one of items 13 to 27, wherein the surfactant is polyoxyethylene-polyoxypropylene co-polymer or block co-polymer, polyoxyethylene sorbitan fatty acid ester, polyoxyethylene fatty acid ester, poly-oxyethylene alkyl ether, sodium alkyl sulfate or sulfonate, sodium alkyl aryl sulfonate, water soluble tocopheryl polyethylene glycol succinic acid ester, polyglycerol fatty acid ester, alkylene polyol ether or ester, polyethylene glycol glyceryl fatty acid ester, sterol, transesterified and polyoxyethylated caprylic-capric acid glyceride, sugar fatty acid ester, PEG sterol ether, phospholipids, salts of a fatty acid, fatty acid sulfate or sulfonate, salt of fatty acid, fatty acid sulfate or sulfonate, medium or long-chain alkyl ammonium salt, bile acid or salt thereof, glycolic acid or a salt, polyoxyethylene mono ester of a saturated C10 to C22 fatty acid, or a combination thereof.
30. A pharmaceutical formulation according to any one of items 13 to 29, wherein the surfactant is polyoxyethylene-polyoxypropylene co-polymer or block co-polymer or a water soluble tocopheryl polyethylene glycol succinic acid ester.
31. A pharmaceutical formulation according to any one of items 13 to 30, wherein the surfactant is a water soluble tocopheryl polyethylene glycol succinic acid ester, preferably Vitamin E polyethylene glycol 1000 succinate.
32. A pharmaceutical formulation according to any one of items 13 to 30, wherein the surfactant is polyoxyethylene-polyoxypropylene co-polymer, preferably poloxamer 188.
33. A pharmaceutical formulation according to any one of items 13 to 30, wherein the surfactant is sodium alkyl sulfate, preferably sodium lauryl sulfate.
34. A pharmaceutical formulation according to any one of items 13 to 33, wherein the weight ratio of the surfactant to 40-O-(2-hydroxy)ethyl-rapamycin is from 10:1 to 1:200 by weight, preferably is 1:1 to 1:100 by weight, more preferably 1:2 to 1:8 by weight, particularly between 1:4 to 1:6 by weight.
35. A pharmaceutical formulation according to any one of items 1 to 34, wherein the formulation further comprises crospovidone, croscarmellose sodium or sodium starch glycolate.
36. A pharmaceutical formulation according to any one of items 1 to 35, wherein the formulation comprises crospovidone.
37. A pharmaceutical formulation according to any one of items 13 to 36, wherein the surfactant is vitamin E polyethylene glycol 1000 succinate, poloxamer 188, sodium lauryl sulfate, or combinations thereof.
38. A pharmaceutical formulation according to any one of items 13 to 37, wherein the formulation comprises a layer separating the surfactant from the 40-O-(2-hydroxy)ethyl-rapamycin.
39. A pharmaceutical formulation according to any one of items 1 to 38, further comprising a desiccant.
40. A pharmaceutical formulation according to any one of items 1 to 39, wherein the pharmaceutical formulation is in a form of a pellet comprising a starter core with a diameter of between 100 µm and 1 mm, preferably between 150 and 500 µm, more preferably between 250 and 355 µm.
41. A package comprising at least one pharmaceutical formulation as defined in any one of items 1 to 40, wherein said at least one pharmaceutical formulation is packed in a package sealed against vapor and moisture permeation.
42. A package comprising at least one pharmaceutical formulation as defined in any one of items 1 to 40 according to item 41, wherein the pharmaceutical formulation is further protected against light.
43. A package according to item 41 or 42, which is a blister pack.
44. A package according to item 41 or 42, which is a bottle made mainly or completely of HDPE (high density polyethylene).
45. A package according to any one of items 41 to 43, wherein the formulation is sealed against vapor permeation by forming a foil/foil blister, preferably an aluminium/aluminium blister, or by forming a pack comprising a blister base part and a cover film consisting of aluminium or an aluminium/plastics material composite, and a lower sealing tray, which is formed from an aluminium/plastics material laminate, being sealed against the rear of the blister base part.
46. A package according to any one of items 41 to 45 meeting the USP 671-requirements of highest class.
47. A process for preparing a pharmaceutical formulation according to any one of items 1 to 40, wherein 40-O-(2-hydroxy)ethyl-rapamycin is mixed with pharmaceutically acceptable excipient and formulated in the pharmaceutical formulation.
48. A process for preparing a pharmaceutical formulation as defined in any one of items 1 to 13 or 15 to 40, wherein at least a layer comprising more than 40 wt % of 40-O-(2-hydroxy)ethyl-rapamycin for the first part is provided by mixing a pharmaceutically acceptable excipient and 40-O-(2-hydroxy)ethyl-rapamycin, and the second part is prepared by mixing 40-O-(2-hydroxy)ethyl-rapamycin and pharmaceutically acceptable excipients.
49. A process for preparing a pharmaceutical formulation as defined in any one of items 1 to 13 or 15 to 40, wherein the layer comprising more than 40 wt % of 40-O-(2-hydroxy)ethyl-rapamycin of the first part is deposited in a form of a coating on a core and the second part is deposited as a second coating comprising less than 40 wt % of 40-O-(2-hydroxy)ethyl-rapamycin on the first coating, optionally with additional sub - or top coatings.
50. A process for preparing a pharmaceutical formulation as defined in any one of items 15 to 40, wherein a second layer comprising a surfactant is provided and above the second layer, a first layer comprising 40-O-(2-hydroxy)ethyl-rapamycin is deposited, optionally with a layer separating the a first and the second layer.
51. A process for preparing a pharmaceutical formulation according to any one of items 47 to 50, wherein coatings are deposited on a starter core with a diameter of between 100 µm and 1 mm, preferably between 150 and 500 µm, more preferably between 250 and 355 µm, to prepare a pellet.
52. A pharmaceutical formulation according to any one of items 1 to 40 for use as a medicament.
53. A pharmaceutical formulation according to item 52 for use in the treatment of a tumor disease or in the prophylaxis of organ rejection.
54. A pharmaceutical formulation according to any one of items 1 to 40, wherein the formulation if free of Eudragit L.
55. A pharmaceutical formulation comprising 40-O-(2-hydroxy)ethyl-rapamycin in a first layer and a surfactant in a second layer, wherein the second layer is above the first layer and the surfactant is not poloxamer 188 and TPGS.
56. A pharmaceutical formulation comprising 40-O-(2-hydroxy)ethyl-rapamycin in a first layer and a surfactant in a second layer according to item 55, wherein the first and the second layers are coatings and the second layer is enclosing the first layer.
57. A pharmaceutical formulation comprising 40-O-(2-hydroxy)ethyl-rapamycin according to item 55, further defined according to any one of the items 15 to 40, respectively, either alone or in combination.
58. A pharmaceutical composition according to any one of items 15 to 40, wherein the formulation further comprises a part releasing at least 85 wt % 40-O-(2-hydroxy)ethyl-rapamycin of that part in less than 60 minutes, preferably less than 30 minutes.

Similar to rapamacyn, 40-O-(2-hydroxy)ethyl-rapamycin is a macrolide of low water solubility and a low chemical stability. Mixtures of O-(2-hydroxy)ethyl-rapamycin with many conventional pharmaceutical excipients can lead to instability; disadvantages with such compositions include unpredictable dissolution rates or irregular bioavailability. 40-O-(2-hydroxy)ethyl-rapamycin is also moisture labile and sensitive to light and oxidative stress.

Thus, specific measurements are required to stabilize the drug substance during processing and throughout the shelf life time of the drug product. Due to chemical instability and low water solubility of the compound it is difficult to formulate it into a galenic composition. Its utility as a pharmaceutical is thus restricted. In addition, 40-O-(2-hydroxy)ethyl-rapamycin has very low and variable bioavailability. When administered orally to humans, solid O-(2-hydroxy)ethyl-rapamycin may not be absorbed in sufficient amount into the blood stream. Solubility enhancing principles have to be applied for ensuring consistent, reliable drug absorption with low variability and degradation of O-(2-hydroxy)ethyl-rapamycin in the gastro-intestinal tract needs to be minimized for optimizing the drug efficacy and for reducing variability of absorption in and/or among patients.

40-O-(2-hydroxy)ethyl-rapamycin is available in solid dosage forms for oral administration as 0.1 to 10 mg immediate release tablets. However, still today it is difficult to formulate 40-O-(2-hydroxy)ethyl-rapamycin as an oral solid dosage form which meets both requirements of satisfying drug product stability and sufficient oral bioavailability at the same time.

Surprisingly we found that the bioavailability of the everolimus can be increased for oral dosage form without impairing the stability of either the final dosage form or the active ingredient itself by providing a pharmaceutical formulation comprising a first part and a second part, wherein the first part comprises a layer with a high dosage load, i.e. more than 40 wt % of 40-O-(2-hydroxy)ethyl-rapamycin, and the second part exhibits immediate release characteristics, i.e. it releases more than 85 % of 40-O-(2-hydroxy)ethyl-rapamycin of the second part in less than 60 minutes. The high drug load by itself slows release of the 40-O-(2-hydroxy)ethyl-rapamycin and causes the first part to release the drug in a sustained release profile. In alternative, the effect can be achieved by providing a pharmaceutical formulation comprising 40-O-(2-hydroxy)ethyl-rapamycin in a first layer and a surfactant in a second layer beneath the first one. Both formulations enable that higher percentage of the active ingredient gets taken up by the body. In addition, specifically designed formulations reduce the need for further excipients that cause deterioration of the active ingredient 40-O-(2-hydroxy)ethyl-rapamycin (everolimus), or keep them separated from the active ingredient. In addition, both alternatives provide a quick onset of drug release.

It has been found that a sustained release can be achieved with a high drug load in the layer containing active ingredient. In this case a separate extended release coating is not needed.

A high drug load as used herein means that the layer comprises more than 45 wt %, more than 50 wt %, more than 60 w t%, more than 70 wt %, more than 80 wt %, or more than 90 wt % of 40-O-(2-hydroxy)ethyl-rapamycin. In a preferred embodiment the layer comprises more than 80 wt % 40-O-(2-hydroxy)ethyl-rapamycin. High drug load of poorly soluble everolimus, preferably in amorphous form, leads to slow release profile. The formulation can thus contain less pharmaceutical excipients of other types, like for example fillers, binders, or pH modifiers and plasticizers in a separate coating. This is particularly advantageous as due to everolimus' chemical instability, everolimus shows in general only a moderate compatibility to any excipient. The drug release can be slow down by reducing the amount of the hydrophilic excipients in the everolimus layer. However, as discovered, the slow release profile may be further advantageously supplemented by immediate release part to further increase the bioavailability of the drug. Therefore, in order to boost bioavailability even higher, the part with the high drug load layer is complemented with a formulation part that also contains 40-O-(2-hydroxy)ethyl-rapamycin, but releases the drug with the immediate release profile. This way, the formulation shows a relatively fast onset of drug release and is capable of sustaining the release over prolonged time. The weight ratio of 40-O-(2-hydroxy)ethyl-rapamycin in the first and the second part, i.e. in the high drug load layer and the immediate release part is from 2:5 to 20:1, preferably is from 5:1 to 20:1. The weight ratio can also be from 8:1 to 12:1, or specifically is 10:1. This achieves that the drug is not lost in the initial burst due to its instability and provides enough drug to sustain a well-balanced release profile. It has been found that a pharmaceutical formulation with a first part and a second part, wherein the first part comprising a high drug load is combined with a fast release second part, provides an unexpected balance between stability and advantageous bioavailability.

The term "Immediate release" as used herein refers to a pharmaceutical formulation which releases 85% of the active drug substance within less than 60 minutes in accordance with the definition of "Guidance for Industry: "Dissolution Testing of Immediate Release Solid Oral Dosage Forms" (FDA CDER, 1997). Specifically the term "immediate release" means release of more than 80% or 90 % everolimus from the formulation within the time of 30 minutes. For example, the release can be measured in a dissolution assay, where a dissolution vessel is filled with 900 mL phosphate buffer pH 6.8 containing sodium dodecyl sulfate 0.2 wt% at 37°C and the dissolution is performed using a paddle method at 75 rpm according to USP by according to USP testing monograph 711, and Ph.Eur. testing monograph 2.9.3. respectively. In alternative, the release can be measured in a dissolution assay, where a dissolution vessel is filled with 900 mL phosphate buffer pH 4.5 at 37°C or 900 mL phosphate buffer pH 6.8 containing 0.06 wt% sodium dodecyl sulfate at 37°C, in both cases performed using a paddle method at 75 rpm according to USP <711>, and Ph.Eur. 2.9.3. respectively. The release can be detected for example with a UV photometer or with HPLC. The term "extended release" can be interchangeably used with the terms "sustained release" or "prolonged release". The term "extended release" relates to a pharmaceutical formulation that does not release active drug substance immediately after oral dosing but over an extended in accordance with the definition in the pharmacopoeias Ph. Eur. (7th edition) monograph for tablets and capsules and USP general chapter <1151> for pharmaceutical dosage forms. The release can also be determined by the aforementioned dissolution assay.

In a specific embodiment, the extended release according to the present disclosure typically denotes release of 40-O-(2-hydroxy)ethyl-rapamycin in the in-vitro release assay according to following release specifications:
0.5h: <45%, or <40, preferably: <30%
1 h: 20-80%, preferably: 30-60%
2h: >50%, or >70%, preferably: >75%
3h: >60%, or >65%, preferably: >85%, particularly >90%.

In one embodiment, where the release is measured in a dissolution assay with 900 mL phosphate buffer pH 4.5 at 37°C as described above, a pharmaceutical formulation according to the present disclosure can exhibit dissolution according to the following release specifications:
0.5h: <10%, preferably <6%
1 h: <12%, preferably <8%
2h: <14%, preferably <12%
3h: <16%, preferably <14%, particularly if the formulation comprises a extended release coating as defined herein; or
0.5h: <20%, preferably <15%
1 h: <30%, preferably <20%
2h: <40%, preferably <30%
3h: <50%, preferably <40%, particularly if the formulation only comprises protective coating and is without extended release coating.

In another embodiment, where the release is measured in a dissolution assay with 900 mL phosphate buffer pH 6.8 containing 0.06 wt% sodium dodecyl sulfate at 37°C as described above, a pharmaceutical formulation according to the present disclosure can exhibit dissolution according to the following release specifications:
0.5h: <40% or <30%, preferably <20%
1 h: >10% or >15%, preferably >20%; 20-60%, more preferably 20-40%
2h: 30-80%, preferably 40-80%
3h: >60%, preferably >70%, particularly if the formulation comprises a extended release coating as defined herein; or
0.5h: >50% or >60%, preferably >65 %
1 h: >80% or >90%, preferably >95%, particularly if the formulation only comprises protective coating and is without extended release coating.

The high drug load part of the formulation, or a pharmaceutical formulation comprising 40-O-(2-hydroxy)ethyl-rapamycin in a first layer and a surfactant in a second layer, wherein the second layer is beneath the first layer, with additional extended release coating in accordance with the present disclosure typically release 50% of the 40-O-(2-hydroxy)ethyl-rapamycin not earlier than 45, 60, 75, 90, 105 min or 120 min in said in-vitro dissolution assay.

The formulation with a high drug load in the layer can also contain a surfactant. It can be added to any layer, preferably to the layer different from the one containing the active ingredient. However, as the case may be, a specially selected surfactant like for example Vitamin E polyethylene glycol 1000 succinate (TPGS) can protect the active ingredient. Therefore, the embodiments with the surfactant in the layer that contains active ingredient are also encompassed herein. The layers can be in a form of a coating. The layer with the surfactant can be beneath the layer that contains the active ingredient. In the event that layers take the form of a coating, the coating comprising the surfactant can be enclosed at least by a coating that comprises everolimus. In addition, further coatings like for example extended release coating or protective coating can be applied over it.

As an alternative solution to deal with poor bioavailability and sensitive chemical nature of the drug we provide a pharmaceutical formulation comprising 40-O-(2-hydroxy)ethyl-rapamycin in a first layer and a surfactant in a second layer, wherein the second layer is beneath the first layer. The release rate of the active ingredient from the formulation is modulated in the presence of the surfactants for example by affecting the speed of water uptake into the layers containing the surfactant and thus leading to disintegration of the formulation or solubilisation of the active ingredient. The surfactant can further mobilize and stabilize the active substance. In addition, the surfactant in the layer different from the active ingredient layer facilitates dissolution of the everolimus and enables advantageous release characteristics of the formulation. At the same time it minimizes the effect of the surfactant on the stability of everolimus. The latter can be further safeguarded by applying an intermittent layer between the surfactant layer and layer comprising the active ingredient. In one embodiment the first layer and the second layer are in a form of a coating. The surfactant can be placed in a coating beneath the coating with everolimus. In such a case the coating that comprises surfactant can be enclosed at least by the coating with everolimus. Further coatings can be deposited over the coating that comprises surfactant. For example, protective layers or sustained release layers as define herein can present further layers of the pharmaceutical formulation.

There can be an additional layer between the first and the second layer that separates them and protects the everolimus from surfactant or other excipients in the surfactant layer. This separation prevents an intimate contact of the surfactant with the active ingredient. The surfactant or wetting agent containing layer may further comprise matrix formers, typically matrix forming polymers, and may contain additional excipients, such as fillers, e.g. lactose, mannitol, maltodextrine, pregelatinized starch, calcium phosphate, or microcrystallline cellulose, and disintegrants, e.g. corn starch, croscamellose, sodium starch glycolate, or crospovidone, antioxidants, e.g. butylhydroxy anisol, butylhydroxy toluol, ascorbyl palmitate, tocopherol, and process enhancing agents, such as lubricants and glidants, e.g. colloidal silicon dioxide, talc, glyceryl monostearate, magnesium stearate, calcium stearate, or sodium stearyl fumarate. Suitable matrix forming polymers used for fast dissolving or disintegrating carrier matrices are known in the art include for instance cellulose or starch, for instance micro-crystalline cellulose ("MCC"), for example Avicel PH 101 (FMC BioPolymer), acacia, sodium alginate, gelatine, starch, pregelatinised starch, methylcellulose, hydroxypropyl methylcellulose ("HPMC"), hydroxypropylcellulose, hydroxyethylcellulose, polyethylene glycol or polyvinylpyrrolidone ("PVP"), carrageenan, such as Gelcarin GP 812 or combinations thereof. The same excipients can also be used to prepare the layer with the high drug load of the aforementioned embodiments.

The term "surfactant" can be used interchangeably with a "wetting agent" or "detergent" and as used herein means a non-ionic, ionic, anionic, cationic or amphoteric surfactant, particularly a non-ionic, ionic, anionic, or amphoteric surfactant. Examples of suitable surfactants/wetting agents include polyoxyethylene-polyoxypropylene co-polymers and block co-polymers known, for example, under the trademarks Pluronic or Poloxamer (e.g. poloxamer 188 (Pluronic F68), polyoxyethylene, sorbitan fatty acid esters including mono and tri lauryl, palmityl, stearyl and oleyl esters of the type known under the trade name Tween, polyoxyethylene fatty acid esters including polyoxyethylene stearic acid esters of the type known under the trade name Myrj, poly-oxyethylene alkyl ethers known under the trade mark Brij, sodium alkyl sulfates like Soldium lauryl sulphate (SDS) and sulfonates, and sodium alkyl aryl sulfonates, water soluble tocopheryl polyethylene glycol succinic acid esters (TPGS), polyglycerol fatty acid esters, alkylene polyol ethers or esters, polyethylene glycol glyceryl fatty acid esters, sterols and derivatives thereof, transesterified, polyoxyethylated caprylic-capric acid glycerides, sugar fatty acid esters, PEG sterol ethers, phospholipids, salts of fatty acids, fatty acid sulfates and sulfonates, salts of fatty acids, fatty acid sulfates and sulfonates, medium or long-chain alkyl, e.g. C6-C18, ammonium salts, bile acid or salt thereof; for example cholic acid, glycolic acid or a salt, e.g. sodium cholate and polyoxyethylene mono esters of a saturated C10 to C22 fatty acid.
In a preferred embodiment the surfactant is polyoxyethylene-polyoxypropylene co-polymer or block co-polymer, or a water soluble tocopheryl polyethylene glycol succinic acid ester, more preferably is a water soluble tocopheryl polyethylene glycol succinic acid ester, particularly is preferably Vitamin E polyethylene glycol 1000 succinate (TPGS). Particularly TPGS shows a surprising power to protect everolimus even in the presence of water. Therefore it is particularly beneficial for the stability of everolimus.
In another embodiment the surfactant in the present pharmaceutical formulation is polyoxyethylene-polyoxypropylene co-polymer, preferably poloxamer 188.
In yet another embodiment, the pharmaceutical formulation according to the present disclosure comprises the surfactant sodium alkyl sulfate, preferably sodium lauryl sulfate.

The surfactant or wetting agent is present in a formulation in a ratio to 40-O-(2-hydroxy)ethyl-rapamycin from 10:1 to 1:200 by weight. In a more preferred embodiment the surfactant ratio to 40-O-(2-hydroxy)ethyl-rapamycin is 1:1 to 1:100 by weight. In another embodiment, the ration of surfactant to 40-O-(2-hydroxy)ethyl-rapamycin can be 1:2 to 1:8 by weight, particularly between 1:4 to 1:6 by weight.

In a special embodiment a pharmaceutical formulation comprising 40-O-(2-hydroxy)ethyl-rapamycin in a first layer and a surfactant in a second layer is provided, wherein the second layer is above the first layer. In this case the surfactant is not poloxamer 188 and TPGS. The surfactant or wetting agent in a second layer can form a protection layer which separates the active ingredient containing layer from the coating covering the formulation. The coating covering the formulation may be an extended release coating.

The pharmaceutical formulations of the present disclosure satisfy product stability requirements and have favourable pharmacokinetic properties over the currently available immediate release tablets, such as reduced average plasma peak concentrations, reduced inter- and intra-patient variability in the extent of drug absorption and in the plasma peak concentration, reduced Cₘₐₓ/ Cₘᵢₙ ratio and reduced food effects. The improved solid formulation of the present invention allow for more precise dose adjustment and reduce frequency of adverse events such as for example stomatitis thus providing safer treatments for 40-O-(2-hydroxy)ethyl-rapamycin to the patients.

The pharmaceutical formulation of the present invention can be in a form of a minitablet, pellet, microparticle, microcapsule, granule, bead, tablet, or a double or multilayer tablet. In a preferred embodiment, the first part of the formulation with a first part containing a high drug load layer and a second immediate release part is in a form of a minitablet, pellet, microparticle, microcapsule, granule, bead, tablet, or a layer of a double or multilayer tablet. The final dosage form can also be prepared having the first part in the form of a pellet or a microcapsule, and the second part in the form of a minitablet or tablet. Both parts can take a form of a layer in a multicoated pharmaceutical formulation, for example a coated bead, a coated pellet or a coated microcapsule. In this case the layer with the high drug load, i.e. a layer with above 40 wt% of active ingredient would be a first coating. This could then be coated with a second coating, which exhibits immediate release characteristics. For example, immediate release can be achieved by preparing a coat that comprises less than 40 wt % of everolimus, preferably less than 20 wt%. In a specific embodiment the formulation would comprise at least a double coated core, wherein one coat comprises more than 40 wt % of everolimus, preferably between 50 wt% and 85 wt%, more than 50 wt%, 60 wt%, and the second coat comprises less than 40 wt % of everolimus, preferably less than 20%.

Both alternatives of the present disclosure, particularly the pharmaceutical formulation comprising 40-O-(2-hydroxy)ethyl-rapamycin in a first layer and a surfactant in a second layer, wherein the second layer is beneath the first layer, can also be in a form of a multiparticulate system.

In one embodiment of the present disclosure the pharmaceutical formulation has further functional layers and coatings. Even the formulation containing high drug layer can be coated or contain additional functional coatings. One possible coating can be for example extended release coating or a protection coating. Beside with the high drug load, a formulation can be prepared to enable release of 40-O-(2-hydroxy)ethyl-rapamycin over an extended period of time, e.g. over at least 1, 2, 3, 4, 5 or 6 hours by using pharmaceutically acceptable excipients, or preparing matrix or a coating that allow extended release.

40-O-(2-hydroxy)ethyl-rapamycin in a pharmaceutical formulation can be formulated in a carrier matrix comprising matrix formers, typically matrix forming polymers, and may contain additional excipients, such as fillers, e.g. lactose, mannitol, maltodextrine, pregelatinized starch, calcium phosphate, or microcrystallline cellulose, and disintegrants, e.g. corn starch, croscamellose, sodium starch glycolate, or crospovidone, antioxidants, e.g. butylhydroxy anisol, butylhydroxy toluol, ascorbyl palmitate, and process enhancing agents, such as lubricants and glidants, e.g. colloidal silicon dioxide, talc, glyceryl monostearate, magnesium stearate, calcium stearate, or sodium stearyl fumarate. The term "matrix former" typically relates to a pharmaceutically inert material which provides physical stability, such as e.g. mechanical or binding stability.

Suitable matrix forming polymers used for the carrier matrix are known in the art and can include for instance cellulose or starch, for instance micro-crystalline cellulose ("MCC"), for example Avicel PH 101 (FMC BioPolymer), acacia, sodium alginate, gelatine, starch, pregelatinised starch, methylcellulose, hydroxypropyl methylcellulose ("HPMC"), hydroxypropylcellulose, hydroxyethylcellulose, polyethylene glycol or polyvinylpyrrolidone ("PVP"), carrageenan, such as Gelcarin GP 812 or combinations thereof. Further suitable matrix forming excipients for carrier matrix, that may further provide extended release properties are known in the art and include for instance acacia, sodium alginate, gelatine, carboxmethylcellulose sodium, (or "CMC sodium"), methylcellulose, ethylcellulose and cellulose acetate or polyacrylates, e.g. ammonio methacrylate copolymers (Eudragit RS/RL), hydroxypropyl methylcellulose ("HPMC"), hydroxypropylcellulose, hydroxyethylcellulose, polyvinylacetate, polyethylene glycol or polyvinylpyrrolidone ("PVP"), e.g. carrageenan, such as Gelcarin GP 812, glyceryl monostearate, stearylalcohol, stearic acid, glyceryl behenate, Vitamin E polyethylen glycol succinate, or combinations thereof.

The coating polymer can be any polymer used in the field for coating the pharmaceutical formulations, like for example hydroxypropylmethyl cellulose. It can be a water soluble polymer. In one embodiment, the coating is formed with a polymer that shows pH independent water solubility. It may also be water insoluble or non-disintegrating polymer. The coating, particularly extended release coating may also contain water soluble excipients, plasticizers, and processing enhancing agents, such as lubricants and anti-tacking agents. The coating, e.g. the extended release coating, typically has a coating thickness in the range of 10 to 100 µm, preferably 10 to 50 µm (assessed by confocal RAMAN spectroscopy).

Suitable extended release coating forming polymers which enable diffusion controlled release are known in the art and include for instance a cellulose ether, polymethacrylate, polyvinylacetate or a combination thereof. The polymers can be ethylcellulose and cellulose acetate or polyacrylates, e.g. ammonio methacrylate copolymers (Eudragit RS/RL), polyvinylacetate or combinations thereof. In a preferred embodiment, the extended release coating forming polymer is ethylcellulose or cellulose acetate or polyacrylates, e.g. ammoniomethacrylate copolymer Type A (Eudragit RS) or ammonio-methacrylate copolymer Type B (Eudragit RL) or combinations thereof. The most preferred is ethyl cellulose. The release mechanism provided by ethyl cellulose is based on pH independent swelling. Moreover, the extended release coating can includes plasticizer, such as triacetine, triethyl citrate, dibutylsebacate, diethylsebacate, polyethylene glycol 3000, 4000 or 6000, acetyltriethylcitrate, acetyltributylcitrate, or diethylphthalate, and/or anti-tacking agents such Syloid 244 FP, talc, glyceryl monostearate, or titanium dioxide. The amount of plasticizer is typically between 5 to 40%, preferably 10 to 25%, relative to the amount of extended release polymer. In a preferred embodiment the composition is free of tryethyl citrate or Eudragit L, as both disturb the stability of everolimus. The active ingredient is particularly not compatible with the two excipients.

Polymethacrylates have the following structure of formula (I): 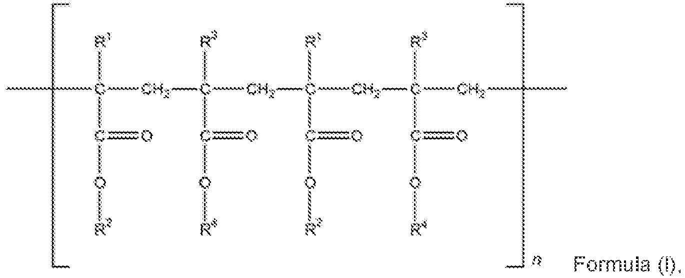
Wherein for Eudragit E, R¹, R³ is CH₃, R² is CH₂CH₂N(CH₃)₂, R⁴ is CH₃, C₄H₉;
for Eudragit L and (Eudragit S, R¹, R³ is CH₃, R² is H, R⁴ is CH₃;
for Eudragit FS, R¹ is H, R² is H, CH₃, R³ is CH₃, R⁴ is CH₃;
for Eudragit RL and Eudragit RS R¹ is H, CH₃, R² is CH₃, C₂H₅, R³ is CH₂, R⁴ is CH₂CH₂N(CH₃)₃ ⁺Cl⁻;
for Eudragit NE 30 D and Eudragit NE 40 D R¹, R³ is H, CH₃, R², R⁴ is CH₃, C₂H₅.

In the extended release coating, in accordance with one preferred embodiment of the present invention, a water soluble or gellating excipients can be added. Preferably, the excipient is readily water soluble excipient. This allows the excipient to facilitate dissolution by introducing pores in the coating and eventually increasing permeability of the coating.

Suitable water soluble compounds for this purpose are known in the art. For example they are hydroxypropylcellulose (HPC (e.g. Klucel™ EF, EXF,LF), or hydroxypropyl methylcellulose (HPMC, e.g. Methocel™ E3/E5, Pharmacoat 603™), polyethylen glycol (e.g. Macrogol 1500, 3500, 4000, 6000), poloxamer 188 (Pluronic F68™) or povidone (PVP, e.g. Kollidon K25/K30), a saccharide, e.g. a monosaccharide, such as dextrose, mannose, fructose, a disaccharide, such as sucrose or glucodifructose or combinations thereof. Preferably the pore former is hydroxypropylcellulose (HPC (e.g. Klucel™ EF, EXF,LF), or hydroxypropyl methylcellulose (HPMC, e.g. Methocel™ E3/E5, Pharmacoat 603™), polyethylen glycol (e.g. Macrogol 1500, 3500, 4000, 6000), polyoxyethylene-polyoxypropylene co-polymer, (e.g. poloxamer 188) or povidone (PVP, e.g. Kollidon K25/K30) or combinations thereof. Suitable amounts of pore formers included in coating are equal to ratios of coating polymer to pore former of e.g. 100:20 to 100:50, or 100:20 to 100:100, preferably ratios of 100:35 to 100:45, particularly ratios of 100:35 to 100:50 relative to the amount of coating forming polymer. Further ratios of the coating polymer and pore former are possible. For example a coating polymer and a pore former can be used in a ratio of coating polymer to pore former of e.g. 100:40 to 100:80, or of e.g. 100:50 to 100:70, specifically ratios of 100:70 or 100:55 can be used. Suitable amounts of coating forming polymers included are equal to percentages of polymer weight increase of e.g. 4% to 15%, 5% to 15%, preferably 5% to 12%, more preferably 6% to 12% weight of total weight of pharmaceutical formulation. A weight gain of about 20% of the total weight of a pharmaceutical formulation can be achieved when the extended release coating is applied to the formulation.

The excipient for preparing the formulation containing the active ingredient can also be sodium alginate, polyacrylic acids (or "carbomers"), carboxmethylcellulose sodium, (or "CMC sodium"), methylcellulose, ethylcellulose and cellulose acetate or polyacrylates, e.g. ammonio methacrylate copolymers (e.g. Eudragit RS/RL), hydroxypropyl methylcellulose ("HPMC") of different viscosity grades (i.e. average polymer chain lengths) and combinations thereof, e.g. Methocel™ CR grades, hydroxypropyl cellulose, e.g. Klucel™ HF/MF, polyoxyethylene, e.g. Polyox™ or polyvinylpyrrolidone ("PVP"), e.g. PVP K60, K90, carrageenan, such as Viscarin™ GP-209/GP-379, or combinations thereof. These excipient have the tendency of further regulating the dissolution by diffusion. Specifically adapting the combination of the excipients can allow adjusting the dissolution rate of the active ingredient according to the need.

Alternatively, the non-disintegrating extended release matrix is formed with excipients, which enable release of the active ingredient by a controlled erosion. The erosion controlled matrices may contain lipophilic matrix formers, and also further excipients, such as fillers, disintegrants and process enhancing agents, such as lubricants and glidants. Lipophilic matrix forming excipients related to this matrix type include lipophilic excipients, such as glyceryl monostearate, e.g. Cutina GMS or Cutina RH, glyceryl behenate, e.g. Compritol 888 ATO, stearyl alcohol, stearic acid, hart fat, e.g. Gelucire™, or Vitamin E polyethylen glycol succinate, e.g. Speziol TPGS or combinations thereof.

Suitable binders, fillers or further excipients include for instance mannitol, pregelatinized starch, microcrystalline cellulose, lactose, calcium phosphate, talc, titanium dioxide, triethylcitrate, Aerosil, antioxidants such as e.g. BHT, desiccants and disintegrant such as e.g. crospovidone or sodium starch glycolate, starch, or croscarmellose.

In accordance with a further aspect of the present invention, the present invention contains strongly hygroscopic excipients, which are able to bind water moisture enclosed in the formulation working as an internal desiccant. Adsorbents such as e.g. crospovidone, croscarmellose sodium, sodium starch glycolate, or starch can be used.

In a preferred embodiment methods to stabilize 40-O-(2-hydroxy)ethyl-rapamycin using crospovidone are provided. Crospovidone is known and widely used as tablet disintegrant. It has surprisingly been found in accordance with the present invention that crospovidone protects 40-O-(2-hydroxy)ethyl-rapamycin from moisture induced degradation. Thus, the present invention provides a method to reduce or prevent moisture induced degradation of 40-O-(2-hydroxy)ethyl-rapamycin using 2% to 25% crospovidone. The crospovidone is part of the powder mixtures used for wet and melt extrusion, part of the powder blend for compressing the minitablets, part of powder blend for tabletting the multiparticulates, are directly added to the multiparticulates in a sachet or capsule filling process. In a related embodiment, the present invention provides the use of crospovidone as internal desiccant for pharmaceutical formulations comprising 40-O-(2-hydroxy)ethyl-rapamycin.

In one aspect, the present invention provides O-(2-hydroxy)ethyl-rapamycin containing particles (0.1 to 0.5 mm), beads, pellets (0.2 to 2 mm) or mini-tablets (1.5 to 3 mm), with a low water moisture content of less than 5 wt% in total or even more preferred with less than 3wt % or less than 2.5wt % in total.

In another aspect, the pharmaceutical formulation of the present disclosure contains strongly hygroscopic excipients that are able to bind water moisture enclosed in the formulation working as an internal desiccant. Adsorbents such as e.g. crospovidone, croscarmellose sodium, sodium starch glycolate, starch can be used, preferably crospovidone, croscarmellose sodium, sodium and starch glycolate. The excipients that reduce water activity in the final formulation are especially beneficial as they reduce the hydrolysis rate of everolimus to a minimum. In a separate embodiment crospovidone is used, as it further stabilizes the formulation.

Said coating polymers, or other excipients mentioned herein can be used to prepare a layer with the high drug load, the immediate release part, the pharmaceutical formulation comprising 40-O-(2-hydroxy)ethyl-rapamycin in a first layer and a surfactant in a second layer, wherein the second layer is beneath the first layer, or the pharmaceutical formulation comprising 40-O-(2-hydroxy)ethyl-rapamycin in a first layer and a surfactant in a second layer, wherein the second layer is above the first layer and the surfactant is not poloxamer 188 and TPGS. Standard formulation techniques can be used to prepare the embodiments of the present disclosure and use them separately or combined in a single formulation. As an example the part with the high drug load layer can be in a form of a coated beads, wherein the coat contains at least 40 wt% of the active ingredient. Coated beads can be filled in a capsule together with an immediate release part that can be in a form of coated beads itself, but can be also in a form of a minitablet or a tablet. In alternative, the high drug load part and immediate release part can be prepared in a form of two coatings of the same coated beads, which can be filled in a capsule. The combination of a high drug load tablet and immediate release tabled is also contemplated by the present disclosure. Another variant of a pharmaceutical formulation can be prepared, where the part with the high drug load layer and an immediate release part are two layers of a double or multilayer tablet. A further variant of the formulation of the present disclosure is having the high drug load part and an immediate release part as two layers of the same formulation, e.g. a granule, bead, pellet, microcapsule, tablet or the like. By the same token, the pharmaceutical composition with a surfactant in a layer beneath the active ingredient containing layer can be prepared. It can be a multilayered multiparticulate formulation or take the form of a multilayered single unit formulation, like for example multilayered tablet.

The present disclosure provides also two special embodiments. One is a pharmaceutical formulation as discussed above, wherein the formulation if free of triethyl citrate and/or Eudragit L, particularly is free of Eudragit L. Everolimus is highly sensitive to chemical degradation and extremely difficult for handling when mixed with other excipients. In a binary mix it is only moderately compatible with customary excipients, but is particularly not compatible with triethyl citrate or Eudragit L. Both excipient cause instant decrease of the active ingredient content if they are present in the formulation and in intimate contact with the active ingredient.

The other special embodiment is a pharmaceutical formulation comprising 40-O-(2-hydroxy)ethyl-rapamycin in a first layer and a surfactant in a second layer, wherein the second layer is above the first layer and the surfactant is not poloxamer 188 and TPGS. It can be formulated in the same way as the formulation with the surfactant beneath the active ingredient layer, only that it allows for a possibility that as long as the surfactant is poloxamer 188 and TPGS, the surfactant can also be, or only be encaging the active ingredient layer.

The immediate release part of the formulation comprising 40-O-(2-hydroxy)ethyl-rapamycin can be prepared by standard techniques and can include excipient or additive besides 40-O-(2-hydroxy)ethyl-rapamycin. Immediate release part can also be prepared by enclosing less that 40 wt % of everolimus in said part, preferably less than 20 wt%. Suitable excipients can be selected from binder, diluent, lubricant, disintegrant, glidant, alone or in combination. Further useful additives may include, alone or in combination, buffer agents, anti-oxidants, colorants, stabilizers, fillers, plasticizers, emulsifiers, preservatives, viscosity-modifying agents, or flavouring agents, without being limited thereto. The part can for example be formulated in a form of a minitablet or tablet, or a tablet layer by compressing. It can also be a bead, pellet, particle, granule, or the like. Methods like mixing, extrusion, spheronization, spraying or the like can be applied. The immediate release part can be prepared in a form of additional coating that can be applied on the coating comprising a high drug load, optionally with additional coating between the two coating or covering both of them. For example, a bead, granule or other core can be first coated with a layer comprising more than 40 wt % everolimus, preferably between 50 wt% and 80 wt%, and then a second coating can be added, wherein the second coating comprises less than 40 wt % everolimus, preferably less than 20 wt% everolimus. With changing the everolimus content in a coating different release characteristics can be achieved. It was surprising observed that the content of everolimus above 40 wt % causes the coating to exhibit everolimus-like hydrophobic properties and thus the high drug load leads to sustained release profile. On the other hand, if the everolimus content in a coating is below 40 wt%, preferably below 20 wt%, and no excipients that cause modified release are added, then the coating exhibits immediate release characteristics.

In one preferred embodiment of the present invention, the formulations of the present inventions are in form of a multiparticulate delivery system. Multi-particulate drug delivery systems in accordance with the present invention are mainly oral dosage forms consisting of multiple, small discrete dose units. In these systems, the dosage form, of the drug substances such as capsule, tablets, sachet or stickpack, contains a plurality of subunits, typically consisting of tens to hundreds or even up to thousands of spherical particles with diameter of 0.05-2.00mm. Formulations of the size 1.5 - 3 mm, e.g. minitablets, present another alternative of the present invention. The dosage form is designed to disintegrate rapidly in the stomach releasing the multiparticulates. The multiparticulates are spread in the gastro-intestinal lumen and will be emptied gradually from the stomach releasing the drug substance in a controlled manner. Part of the multiparticulate formulation contains a high drug load and the other exhibits immediate release characteristics; or the multiparticulate system contains surfactant in the correct layer to properly steer the dissolution.

In one embodiment the pharmaceutical compositions according to the present invention, e.g. in form of multi-particulate delivery system, comprise a high drug load of O-(2-hydroxy)ethyl-rapamycin as active ingredient, e.g. dissolved or dispersed in the core of the particle, (e.g. a bead, pellet, granule or minitablet), or in a layer surrounding an inert core of the particle. The active ingredient can be for instance be embedded in an extended release matrix, preferably comprising a hydrophilic or lipophilic matrix forming excipients, or embedded in a fast disintegrating and/or dissolving matrix in combination with functional layer(s) and top coating(s) wherein at least one of the functional layer(s) or top coating(s) comprises a coating forming polymer enabling diffusion controlled extended release of the active ingredient. The immediate release part can also be in a form of a particle, (e.g. a bead, pellet, granule or minitablet) containing O-(2-hydroxy) ethyl-rapamycin. In the same manner the pharmaceutical formulation with the surfactant in the coat or underneath can be prepared. Optionally, a protection layer for improving stability of the active ingredient separates the matrix containing the active substance from functional layers or top coatings, to ensure stability of the drug product.

In a another preferred embodiment, the present invention provides stable extended release formulations, e.g. in form of a multiparticulate delivery system, comprising 40-O-(2-hydroxy)ethyl-rapamycin as active ingredient, a layer containing surfactant beneath the active ingredient, or in alternative above the active ingredient, wherein then the surfactant is not poloxamer 188 and TPGS and an outer coating layer comprising a pH independent water soluble polymer and a water soluble component as a pore former, and optionally further functional layers like for example protective layer.

In a preferred embodiment, the pharmaceutical composition of the present invention contains 40-O-(2-hydroxy)ethyl-rapamycin as sole therapeutically active ingredient.

In one preferred embodiment, the particles comprise one or several top coats enabling extended release of the active ingredient. Top coats typically are final layers with release controlling behaviour, which are enclosing each particle of the multiparticulates separately.

In a particularly preferred embodiment, the pharmaceutical composition of the present invention with the surfactant beneath the active ingredient layer, or in alternative above the active ingredient, wherein then the surfactant is not poloxamer 188 and TPGS, comprise an outer layer or a top coating that controls the release by the diffusion of the drug through the coating layer which is permeable, optionally by the formation of pores in the insoluble polymer layer, or alternatively solely by the hydration of the insoluble polymer, or that controls the release by a combination of a pore former and hydration of the insoluble polymer. The polymer is insoluble independently from pH, and optionally contains water soluble pore former. The release rate is affected by the extent of pore formation after the pore former is dissolved. The insoluble coating polymer can be cellulose ethers such as ethylcellulose and cellulose acetate or polyacrylates, e.g. ammonio methacrylate copolymers (Eudragit RS/RL). Suitable pore formers include water soluble cellulose ethers, for instance hydroxypropylcellulose (HPC (Klucel™ EF, EXF, LF) or hydroxypropyl methylcellulose (HPMC, Methocel™ E3/E5, Pharmacoat 603™), polyethylen glycol (Macrogol 1500, 3500, 4000, 6000), poloxamer 188 (Pluronic F68™) or povidone (PVP, Kollidon K12, K25, K30). For instance, water soluble pore former can be mixed with insoluble polymer in a ratio of 2:1 to 1:10, e.g. 1:1 to 1:5, 1:3 or 1:5. A preferred pore former to insoluble polymer ratio in accordance with the present invention is HPC, e.g. Klucel™ EF,EXF,LF or HMPC 3cP, e.g. Methocel™ E3, in a ratio of 1:1 to 1:4, e.g. about 1:1, 1:1.2, 1:1.5 or 1:2. The preferred insoluble polymers in accordance with the present invention are ethylcellulose (EC, Aqualon EC N10™) in combination with a pore former. Without the use of a pore former, preferably the combination of the insoluble polymers ammoniomethacrylate copolymer Type A (Eudragit RS) and ammonio-methacrylate copolymer Type B (Eudragit RL) at ratios of 1:2 to 9:1, preferably 1:1 to 4:1 are applied in accordance with this invention.

The sustained release top coat(s) preferably achieve release of majority of the active substance into the small intestine and allows protecting the active substance from stomach fluids and minimizes the exposure of the active substance to the mouth, oesophagus and stomach. The same is achieved with the formulation according to the present disclosure that uses only high drug load to extend the release profile of O-(2-hydroxy)ethyl-rapamycin. To all of these embodiments, it is preferred to add part of the formulation with immediate release characteristic. Combined they achieve advantageously flat and extended release profile without too high plasma peak concentrations and low cₘₐₓ/cₘᵢₙ ratio.

In one embodiment, the pharmaceutical formulation according to the present disclosure, either with a high drug load layer, or a surfactant layer variant, is in a form of a pellet comprising a starter core with a diameter of between 100 µm and 1 mm, preferably between 150 and 500 µm, more preferably between 250 and 355 µm. Starter cores of size below 100 µm are extremely hard to process, particularly on a large scale. The size of starter cores above 1 mm may reduce the surface area to the extent where the formulation no longer exhibits a dissolution profile with a beneficially low cmax/cmin ratio. The starter cores of size between 250-355µm are particularly advantageous. Selecting the starter core of said size allows to prepare the pellets faster with shorter processing times, with less agglomeration during spray drying steps and less electrostatic interference. The coated starter cores of the given size proved to give final pellets a narrow size distribution and an optimal surface area to yield advantageous dissolution profile. Starter core can be for example a sugar sphere, a particle of an inert pharmaceutical excipient or the like. The size of starter cores can be determined for example by a sieve analysis. In alternative, their diameter can be measured by a microscope, where the largest diameter of a core or a particle should fall within the given range.

When a pharmaceutical formulation according to the present disclosure is prepared on a large scale by coating the starter cores, the sheer mass of the material in a production vessel can cause the starter cores to break or chip. Therefore, starter cores can be first coated with a layer of, for example a coating polymer, to stabilize them. In some instances spraying them with water may already give them enough elasticity for further processing. This way breakage or chipping of possibly brittle or friable starting cores can be reduced or prevented.

According to one embodiment of the present invention, the drug substance containing matrix is layered onto the surface of starter cores. The layer can comprise high drug load, i.e. at least 40%, 50%, 60%, 70% or 80% by weight of the layer is O-(2-hydroxy)ethyl-rapamycin. The starter cores could be pre-treated with a layer containing a surfactant. The active ingredient layer is deposited by spraying a dispersion or solution of the matrix components and the drug substance on to particles of uniform, regular size and shape in a fluid bed process. Alternatively, powder mixtures of the matrix components can be layered using a rotating disk processor. Starter cores have an average particle size 0.1 to 2.5 mm. They can be single crystals, e.g. sucrose, or granular agglomerates manufactured by fluid bed granulation, a rotor granulation, extrusion and spheronization, or a compaction process. This encompasses also minitablets that can be used as starter cores. Preferably, the starter cores have a spherical shape and consist of inert material such as sucrose and starch (Sugar Spheres, Suglets™, Non-pareils), mannitol (e.g. MCells™), lactose (e.g. spray dried lactose) or microcrystalline cellulose (e.g. Cellets™). Further extended release or protective layers are applies, as appropriate. Preferably, thus obtained particles can then combined with the immediate release formulation containing O-(2-hydroxy)ethyl-rapamycin.

As a further possibility to prepare the formulation according to the disclosure, the active ingredient containing matrix is incorporated in the cores of the particles. The matrix forming excipients, fillers, and other ingredients for enhancing the process are mixed together with the active ingredient. The content of the active ingredient in the mixture is at least 40 wt%. The powder mixtures obtained can be formulated as particles by using wet extrusion or melt extrusion and subsequent spheronization, or by compacting the mixtures into minitablets. The matrices formed could be combined with separate fast disintegrating/dissolving matrices, or further non-disintegrating matrices with extended release properties built with hydrophilic or lipophilic matrix forming excipients.

In a one embodiment, multiparticulates consisting of a hydrophilic, non-disintegrating matrix which contains the active ingredient or a solid dispersion thereof, are prepared by mixing the active ingredient, a filler, e.g. lactose, together with hydrophilic, hydrogel forming polymers with different viscosities, a glidant, and a lubricant. The hydrophilic, hydrogel forming polymer is preferably for example hydroxypropyl methylcellulose, with low viscosity grade of less than 20 mPas for a 2% by weight aqueous solution, e.g. Methocel E5, combined with hydroxypropyl methylcellulose grade with high viscosity of more than 100 mPas for a 2% by weight aqueous solution, e.g. Methocel K100. The powder mixture is then compressed on the tabletting machine to obtain minitablets. Alternatively, the powder mixture can be wetted with organic solvent, e.g. ethanol, and then extruded and spheronized for obtaining multiparticulates. The formulation contains high drug load and is combined with the immediate release particles. The latter can be prepared for example by simply mixing the active ingredient with binders and fillers, optionally disintegrants and lubricants. The mixture can be compressed to form a minitablet or a tablet.

O-(2-hydroxy)ethyl-rapamycin is chemically instable and prone to degrade when in contact with incompatible excipients, and in particularly when in contact with water/moisture or oxygen. Consequently, to achieve satisfactory chemical stability of O-(2-hydroxy)ethyl-rapamycin in the pharmaceutical formulation, the excipients that are incorporated in the formulation should be selected and should preferably not contain excipients with acidic properties like for example commonly used pH sensitive polymers that are typically used for enteric coating. Preferably, the formulation does not contain Eudragit L . In order to limit water activity in the formulation an excipient that is able to bind water moisture enclosed in the formulation is preferred. Adsorbents such as e.g. crospovidone, croscarmellose sodium, sodium starch glycolate, starch can be used, preferably crospovidone, croscarmellose sodium, sodium and starch glycolate can be used. To further limit the water content in the formulation a desiccant can be added. The desiccant can be located in primary packaging, which preferably is a very tight material not permeable to water vapor. The desiccant actively participates in controlling humidity to which the drug product is exposed during shelf life.

Another aspect of the present disclosure is to retain advantageous chemical stability during extended storage times. This can particularly be achieved when the pharmaceutical formulation according to the present disclosure is packed or saved immediately after production within packages and especially blister packs, bottles or press-through package (PTP) that are essentially or totally impermeable towards water vapor and moisture. More preferably, the whole production is performed under conditions of moisture vapor being at most 50% relative humidity (RH), more preferably at most 20 % RH at 20°C. Suitable packages are essentially or totally water vapor/moisture impermeable and include. The packages are not limited to foil/foil packs such as aluminium/aluminium blister, high density polyethylene (HDPE) bottles, sheets made of plastics having water vapor barrier properties improved such as coated poly(vinyl chloride) or polypropylene, laminated sheets of a polypropylene and a poly(vinylidene fluoride), and blister packs with a - typically thermoformed - blister base part known under the term "tropical blisters". Preferably, the blister packs according to the disclosure have cold-formed foil/foil blister design and further preferably have black base parts and/or covers, allowing up to 100% protection from moisture, oxygen and light. One element of the foil/foil blister pack comprises a lamination of plastic film (e.g. PVC or PE), adhesive, foil, adhesive, and an outer plastic film. The outer film, which can be PET or PVC, supports the thin aluminium layer and acts as the heat-seal layer. The aluminium layer usually consists of several very thin layers rather than a single thick one. The multiple layers help ensure that pinholes do not go all the way through the foil. They also increase the stretchability of the metal and facilitate the cold-stretching process. These multilayer webs are formed, filled, and sealed on a machine that performs these functions in sequence much as the thermoform-fill-seal machine does except that neither web is heated before the forming step. In the process of making the foil/foil blister pack, during the cold-forming process, the foil is shaped and moulded around a plug to form a cavity.

In "tropical blisters", the cover film consists of aluminium or an aluminium/plastics material composite, and a lower sealing tray, which is - typically cold-formed - made from an aluminium/plastics material laminate, is sealed against the rear of the blister base part. Therefore, in a tropical blister, the blister base part with the filling is completely protected by the aluminium films in the cover layer and in the lower sealing tray against the penetration of steam and gases from the external atmosphere.

In a preferred embodiment the package to store the pharmaceutical formulation meets the USP 671-requirements of highest class. The package provides protection from moisture permeation, or oxygen permeation, as specified by the highest standards of the USP 671. The package can be a blister card or a bottle made of foil of polychlortrifluorethylen (PCTFE), polyvinylidenchlorid (PVDC), ethylene vinyl alcohol (EVOH), or combination thereof to satisfy the highest protection standards. Packaging material can be selected based on the overall permeation of water vapor and/or oxygen and combined with different materials to produce a multiple-layer blistering or bottle material with excellent barrier properties against moisture and oxygen. The material can also be combined with aluminium foil. The package can further contain a desiccant to ensure low moisture content in the packaging material.

The pharmaceutical compositions according to the present invention have been found to reduce the peak concentration (Cₘₐₓ) to concentration at 24 hours post-dose (C₂₄ₕ) ratio after a single dose administration in 24 healthy subjects, as compared to the current 40-O-(2-hydroxy)ethyl-rapamycin tablets available to patients (Final Market Image or "FMI" tablets). A typical Cₘₐₓ of the formulations according to the present invention is <10ng/ml. The reduced Cₘₐₓ/C₂₄ₕ ratio, by pharmacokinetic model simulations, is predicted to reduce the Cₘₐₓ to minimum concentration (Cₘᵢₙ) ratio (Cₘₐₓ/Cₘᵢₙ) in a concentration-time profile during a 24-hour dosing interval after daily administration of the present invention. The advantage of the reduced Cₘₐₓ/Cₘᵢₙ ratio of the present invention is that, with the appropriate dose based on the bioavailability of the present invention relative to the formulation currently available on the market, the present invention enables the concentration of everolimus to maintain above the lower therapeutic range of everolimus (for sufficient efficacy) and at the same time distance away from the upper therapeutic range of everolimus (concentration region of toxicity). Thus, the present invention is able to improve the safety profile of everolimus without affecting its efficacy. The pharmaceutical compositions according to the present invention thus allow for instance better exploitation of the therapeutic window of 40-O-(2-hydroxy)ethyl-rapamycin. Typical Cₘₐₓ/C₂₄ₕ (thus typical Cₘₐₓ/Cₘᵢₙ) ratio in patients having administered the pharmaceutical compositions according to the present inventions is < 5 or <4, e.g. 3.5±1 or 3±0.5.

In accordance with one embodiment of the present invention, 40-O-(2-hydroxy)ethyl-rapamycin is contained in a layer made of any substance which is suitable for dispersing or dissolving O-(2-hydroxy)ethyl-rapamycin. In a preferred embodiment, the layer comprising O-(2-hydroxy)ethyl-rapamycin is made of a hydrophilic carrier matrix. The carrier matrix is embedding O-(2-hydroxy)ethyl-rapamycin and protecting it thereby against degradation. Suitable matrix formers are hydrophilic polymers, e.g. HPMC, for example HMPC type 2910 or type 2280, copovidone, HPC, HEC, MEC, MHEC, povidone, which can be dissolved or rapidly dispersed in water. In one preferred embodiment, the matrix layer is in form of a solid dispersion, for instance as described in WO97/03654 or WO03/028705.

In a preferred embodiment, the fast dissolving/disintegrating carrier matrix for 40-O-(2-hydroxy)ethyl-rapamycin is in form of a solid dispersion. The solid dispersion for instance comprises a carrier, e.g. a water-soluble polymer, for example one or a mixture of the following polymers may be used:
- hydroxypropylmethylcellulose (HPMC), e.g. Hypromellose type 2910, which is available as Methocel™ E from Dow Chemicals or Pharmacoat ™ from Shin Etsu. Good results may be obtained using HPMC with a low apparent viscosity, e.g. below 100 cps as measured at 20°C for a 2% by weight aqueous solution, e.g. below 50 cps, preferably below 20 cps, for example HPMC 3 cps;
- polyvinylpyrrolidone (povidone, PVP), e.g. PVP K25, K30 or PVP K12. PVP is available commercially, for example, as Kollidon® from the BASF company or as Plasdone® from ISP company. A PVP having an average molecular weight between about 8,000 and about 50,000 Daltons is preferred, e.g. PVP K30;
- hydroxypropylcellulose (HPC), e.g. Klucel EF/LF/JFor a derivative thereof. Examples of HPC derivatives include those having low dynamic viscosity in aqueous media, e.g. water, e.g. below about 400 cps as measured in a 5 % aqueous solution at 25°C. Preferred HPC derivatives an average molecular weight below about 200,000 Daltons, e.g. between 80,000 and 140,000 Daltons. Examples of HPC available commercially include Klucel® LF, Klucel® EF and Klucel® JF from the Hercules Aqualon company; and Nisso® HPC-L available from Nippon Soda Ltd;
- a polyethylene glycol (PEG). Examples include PEGs having an average molecular weight between 1000 and 9000 Daltons, e.g. between about 1800 and 7000, for example PEG 2000, PEG 4000, or PEG 6000 (Handbook of Pharmaceutical Excipients, p. 355-361);
- a saturated polyglycolised glyceride, available for example, as Gelucire®, e.g. Gelucire® 44/14, 53/10, 50/13, 42/12, or 35/10 from the Gattefossé company; or
- a cyclodextrin, for example a β-cyclodextrin or an α-cyclodextrin. Examples of suitable β-cyclodextrins include methyl-β-cyclodextrin; dimethyl-β-cyclodextrin; hydroxyproypl-β-cyclodextrin; glycosyl-β-cyclodextrin; maltosyl-β-cyclodextrin; sulfo-β-cyclodextrin; a sulfo-alkylethers of β-cyclodextrin, e.g. sulfo-C₁₋₄-alkyl ethers. Examples of α-cyclodextrins include glucosyl-α-cyclodextrin and maltosyl-α-cyclodextrin.

In one preferred embodiment, the O-(2-hydroxy)ethyl-rapamycin-containing layer, contains antioxidant in a ratio of 1:1000 to 1:1 related to the amount of drug substance. The antioxidant may also be present in other functional layers, e.g. at concentration of 0.1 to 10%, preferably 0.1 to 1%. Suitable antioxidants include for instance butyl hydroxyl toluol, butyl hydroxyl anisol, ascorbyl palmitate, tocopherol, vitamin E polyethylene glycol succinate. In a preferred embodiment, the antioxidant is butyl hydroxyl toluol.

In one preferred embodiment, a protection layer separates the layer containing the active substance from other functional layers or parts of the formulation, such as e.g. the top coating or intermittent layer, to enhance stability of the of the drug product. The drug substance is stabilized by excluding any direct contact with the destabilizing excipients. The protection layer also acts as diffusion barrier preventing any components in the top coating, e.g. polymer by-products or plasticizers, which can migrate through the layers, from getting in direct contact with the active. Beside the polymers, which are used also as matrix formers (e.g. the matrix formers described above), high content, of inorganic pigments or antitacking agents such as talc and/or titanium dioxide, e.g. 5 to 100 wt%, or 10 to 100 wt%, preferably 5 to 35 wt%, or 20 to 50 wt%, relative to the applied amount of polymer, contribute to the barrier function. The protection layer thickness can be adjusted to gain optimized drug product stability.

In another preferred embodiment, the active ingredient 40-O-(2-hydroxy)ethyl-rapamycin is directly embedded in the extended release carrier matrix as herein described.

The pharmaceutical compositions of the present invention provide good stability for active substance such as e.g. 40-O-(2-hydroxy)ethyl-rapamycin.

A common side effect O-(2-hydroxy)ethyl-rapamycin formulations is mucositis, more specifically stomatitis, which can lead to additional suffering of the patients, poor patient compliance and suboptimal efficacy. The underlying cause for mucositis is not known and could for instance be due to local irritation of the mucous membranes, but also due to a systemic effects. The formulation of the present invention can reduce or eliminate mucositis as side effect of O-(2-hydroxy)ethyl-rapamycin administration.

The pharmaceutical formulation, e.g. a multiparticulate delivery system of according to the present invention can be formulated into a drug product such as e.g. capsules (e.g. HPMC or Hart Gelatine capsules), or filled into sachets or stick-packs, or formulated as tablets which release the particles upon disintegration.

For further improvement of the drug product stability, the primary packaging, such as sachets, stickpacks, blisters or bottles may include a water adsorbing ingredient, e.g. a silica gel, which is reducing or stabilizing the water moisture content of the drug product during shelf life storage and/or in during in-use time.

The formulation of the present invention may consist of and/or release multiple pellets, granules or minitablets.

Where the pharmaceutical composition of this invention is in a form of a dosage unit, e.g. as a tablet, capsule, granules, each unit dosage will suitably contain between 0.1 mg and 40 mg of the drug substance, more preferably between 1 and 20 mg; for example 0.1, 0.25, 0.5, 0.75, 1.0, 2.0, 2.5, 3.0, 5.0, 10 and 20 mg. Further suitable dosage units include e.g. 25 mg or 30mg or 35 mg or 40 mg or 50 mg. Such dosage units are suitable for administration 1 to 5 times daily depending upon the particular purpose of therapy, the phase of therapy and the like. In one embodiment the unit dosage form is administered once daily. The exact amount of the compositions to be administered depends on several factors, for example the desired duration of treatment and the rate of release of O-(2-hydroxy)ethyl-rapamycin.

The formulations of the present invention have further advantageous properties over currently used formulations. For instance, the formulations of the present invention:
- allow flexible dose adjustments
- allow to meet a tailored drug release profile, e.g. by combining granules, beads, pellets or minitablets with different release profiles (e.g. an initial pulse and sustained release)
- allow to prevent contact of drugs with mucus membrane in the mouth
- allow extended release coated pellets, granules or mini-tablets protect the drug in the stomach against degradation leading to higher bioavailability
- allow extended release profiles
- protect the stomach mucosa against irritation through direct contact with the drug
- lower Cmax and reduce Cmax/Cmin ratio
- reduce inter and/or intra-patient variability in Cmax and AUC
- reduce food dependent inter- and/or intra-patient variability in Cmax and AUC.

Accordingly, in one embodiment, the present invention provides a pharmaceutical formulation or a solid dosage form for use as a medicine. In another embodiment the present invention provides a method for the treatment of mTOR sensitive diseases e.g. as described herein below wherein O-(2-hydroxy)ethyl-rapamycin is administered as 15mg, 20mg, 25mg, 30mg, 35mg, 40mg, 45mg or 50mg dose, e.g. once per day. In a preferred embodiment O-(2-hydroxy)ethyl-rapamycin is administered is administered 1 mg to 40mg, e.g. 20mg to 40mg (e.g. 20mg, 25mg, 30mg, 35mg or 40mg) once per day or 2mg to 80mg, e.g. 20mg to 80mg (e.g. 20mg, 30mg, 40mg, 50mg, 60mg, 70mg or 80mg) every second day or 5mg to 150mg, e.g. 40mg to 150mg (e.g. 40mg, 50mg, 60mg, 80mg, 100mg, 120mg or 150mg) once per week. mTOR sensitive diseases include in particular solid tumor diseases, e.g. renal cell carcinoma, TSC, gastric cancer, breast cancer, lymphoma, hepatocellular cancer.

The drug pharmaceutical compositions according to the present inventions, e.g. multiparticulates formulations, can be prepared either by extruding and spheronizing a mixture of the matrix forming excipients together with the drug substance with the aid of heat or wetting liquids, or by compacting tablets or minitablets with drug containing mixtures, or by layering the drug containing matrix layer onto cores in a fluid bed or rotor granulation process. The layer containing the active substance can be prepared by spraying a spray dispersion with organic solvents in which the hydrophilic components and the active substance are dispersed or dissolved, preferably dissolved, onto the core material, while concurrently the solvents are continuously removed by the aid of heated, dry air. By this process a matrix layer surrounding the cores is formed, more preferably the layer formed is a solid dispersion of the active in polymers such as e.g. HPMC, HPC, HEC. In alternative, the first tablet layer comprising everolimus can be compressed and made into a double layer tablet by compressing another tablet layer on top. It will be apparent that a tablet layer can have a high drug load, or be immediate release layer, or contain surfactant, as the case may be.

Pharmaceutical formulation according to the present inventions can for instance be prepared as follows: a coating containing a surfactant is deposited on inert beads. Then an organic feed mixture for spraying in which the hydrophilic polymer is dispersed in colloidal manner and 40-O-(2-hydroxy)ethyl-rapamycin is dispersed or dissolved, which precipitate together as a uniform, smooth layer of solid dispersion upon removal of the solvent in such a way that they for instance can be further coated with modified release coats.

The obtained drug containing multiparticulates can be coated with additional functional layers and top coatings. A spray dispersion containing coating polymers, lubricants, anti-tack agents, water soluble excipients and plastisizers, which are dissolved, dispersed and suspended in organic solvents and mixtures thereof, is sprayed onto the drug containing multiparticulates. During processing the multiparticulates are kept continuously in a controlled motion or fluidization, while dry, heated process gas is applied to the product bed for evaporating the solvents from the surface of the multiparticulates, where the film layer is formed at a defined temperature. The film layer thickness can be controlled by the amount of coating dispersion sprayed. Final drying is applied for minimizing the residual solvent content in the layered and coated multiparticulates.

The coating process for preparing a pharmaceutical formulation according to current disclosure can be used to obtain about 30 % weight gain when applying the layer comprising 40-O-(2-hydroxy)ethyl-rapamycin, about 20% weight gain when adding a protective layer, about 20% weight with the extended release layer and about 20 % weight gain when coating the surfactant layer over inert beads, which can be optionally further coated. All increases in formulation mass are calculated based on the total weight of the pharmaceutical formulation.

The multiparticulates can be filled into hard capsules, into sachet, stickpacks, or compressed into tablets after mixing them with suitable tabletting agents.
Also provided are treatment methods for mTOR pathway sensitive diseases, such as e.g. described below, by using pharmaceutical composition according to the present invention, e.g. a multiparticulate delivery system.

The oral pharmaceutical compositions of this invention are useful for the treatment or prevention of diseases or conditions responsive to inhibition of mTOR signalling pathway e.g. the following conditions:
a) Treatment and prevention of organ or tissue allo- or xeno-transplant rejection, e.g. for the treatment of recipients of e.g. heart, lung, combined heart-lung, liver, kidney, pancreatic, skin or corneal transplants. They are also indicated for the prevention of graft-versus-host disease, such as following bone marrow transplantation.
b) Treatment and prevention of autoimmune disease and of inflammatory conditions, in particular inflammatory conditions with an etiology including an autoimmune component such as arthritis (for example rheumatoid arthritis, arthritis chronica progrediente and arthritis deformans) and rheumatic diseases. Specific autoimmune diseases for which the compounds of the invention may be employed include, autoimmune haematological disorders (including e.g. haemolytic anaemia, aplastic anaemia, pure red cell anaemia and idiopathic thrombocytopenia), systemic lupus erythematous, polychondritis, scleroderma, Wegener granulamatosis, dermatomyositis, chronic active hepatitis, myasthenia gravis, psoriasis, Steven-Johnson syndrome, idiopathic sprue, autoimmune inflammatory bowel disease (including e.g. ulcerative colitis and Crohn's disease) endocrine ophthalmopathy, Graves' disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis, glomerulonephritis (with and without nephrotic syndrome, e.g. including idiopathic nephrotic syndrome or minimal change nephropathy) and juvenile ermatomyositis.
c) Treatment and prevention of asthma.
d) Treatment of multi-drug resistance (MDR). MDR is particularly problematic in cancer patients and AIDS patients who will not respond to conventional chemotherapy because the medication is pumped out of the cells by Pgp. The compositions are therefore useful for enhancing the efficacy of other chemotherapeutic agents in the treatment and control of multi drug resistant conditions such as multidrug resistant cancer or multi drug resistant AIDS.
e) Treatment of proliferative disorders, e.g. tumors, hyperproliferative skin disorder and the like for instance solid tumors: e.g. Renal Cell Carcinoma, Neuroendocrine tumor e.g. GEP Neuroendocrine Tumors, Phaeochromocytoma, Meningioma, Head and neck squamous cell carcinoma, Breast Cancer, Lymphoma NOS, Thyroid carcinoma NOS Endometrial cancer, Hepatocellular carcinoma, Prostatic Cancer, Metastatic melanoma, Glioma, Glioblastoma Multiforme, Non-small cell Lung Cancer (NSCLC), Mastocytosis, Metastatic Lung Cancer, Hepatocellular carcinoma, Gastrointestinal Stromal Tumours (GIST), Hepatocellular carcinoma, Astrocytoma, Tuberous sclerosis, e.g. SEGA, AML, Lymphangioleiomyomatosis, Thyroid carcinoma NOS, Bile duct Cancer, colorectal Cancer, Adenoid cystic carcinoma, Cholangiocarcinoma, Sarcoma NOS, Mesothelioma, Malignant hepatic neoplasm, colorectal Cancer, Metastatic melanoma, Cervical Cancer, Metastatic Breast Cancer, Bladder Cancer, Non-Hodgkin's lymphoma, Hodgkin's lymphoma, Kaposi's sarcome, Squamous cell carcinoma, Urothelial Cancer, Neoplasm of Digestive Organs, Gastric Cancer, pancreatic Cancer; or liquid tumors: e.g. Advance Hematologic Malignancies, e.g. Leukaemia, e.g. acute myeloid leukaemia, Acute Myeloid Leukaemia, Multiple myeloma.
f) Treatment of abnormally increased bone turnover or resorption, e.g. osteoporosis, bone loss associated e.g. with aromatase inhibitor treatment, rheumatoid arthritis, osteopenia, osteogenesis imperfecta, hyperthyroidism, anorexia nervosa, organ transplantation, joint prosthesis loosening, periarticular bone erosions in rheumatoid arthritis, osteoarthritis, hypercalcemia, bone cancer and bone metastases induced by a primary tumour, multiple myeloma.
f) Treatment of fungal infections.
g) Treatment and prevention of inflammation, especially in potentiating the action of steroids.
h) Treatment and prevention of infection, especially infection by pathogens having Mip or Mip-like factors.
i) Treatment of overdoses of FK-506 and other macrophilin binding immunosuppressants.

Exemplified below are some examples of pharmaceutical formulations comprising 40-O-(2-hydroxy)ethyl-rapamycin that, when administered, allow for advantageous release profile that leads to reduced average plasma peak concentrations, reduced Cmax/ Cmin ratio and shows reduced food effect. The improved release characteristics of the disclosed formulations translate in reducing the incidence of side effects, particularly of mucositis. The formulations provide for increased bioavailability and increased steady-state Cmin, which in turn leads to improved efficacy. In addition, the formulations are more robust and exhibit better physicochemical stability. The beneficial effects of the formulations of the invention can also be determined by other test models known as such to the person skilled in the pertinent art.

### EXAMPLES

### Example 1:

### Protection layered pellets for a dose of 5 mg everolimus:

The following example of drug layered and protection layered pellets provide an immediate release form of mulitparticulates which can be further coated to receive a product with extended release properties. Drug load was adjusted to a percentage, which allowed for filling of 5 mg into capsule size 0. According to the two different compositions described in table 1 different thicknesses of the protection layer was realized to optimize protective effect. A procedure for preparing multiparticulates with a drug containing matrix layer was as follows: The matrix forming polymer HPMC (type 2910, 3 cP) was dispersed in ethanol at a ratio of 4:1 related to the drug substance with a final concentration of 6% in the solvents. Antioxidant butyl hydroxyl toluol was added to the dispersion at an amount equal to 2% related to drug substance. A small fraction of water equal to 6% of total amount of solvents was used for dispersing 7.5% talc and 3.0% titanium dioxide based on solids in the layer with the aid of a homogenizer. The aqueous suspension was added to the dispersion. During continuous stirring the dispersion was equilibrated until the swollen polymer particles were disintegrated. Finally, the drug substance was added and dispersed in the coating dispersion prior to starting the layering onto sugar spheres of 355 to 425 µm, preheated and fluidized in a fluid bed processor. The amount of sugar spheres used resulted in a drug concentration of 1.5% in the active layered multiparticulates after spraying. The spraying occurred at a controlled product bed temperature in the range between 35° and 45°C using a tangential spray process. After finishing the spraying process, when a weight gain of 9.2% was received, the obtained multiparticulates were dried in the fluid bed at temperatures up to 65°C.

A subsequent layering procedure followed for applying a protective, stability enhancing layer: The binding polymer HPMC (type 2910, 3 cP) was dispersed in ethanol with a final concentration of 4% in the solvents. A small fraction of water equal to 6% of total amount of solvents was used for dispersing 25% talc and 5% titanium dioxide with the aid of a homogenizer. The aqueous suspension was added to the dispersion. During continuous stirring the dispersion was equilibrated until the swollen polymer particles were disintegrated. The active layered multiparticulates were preheated and fluidized in a fluid bed processor. The spraying was conducted at a controlled product bed temperature in the range between 35° and 45°C using a bottom spray process until a weight gain of 10 to 15% was received. After finishing the spraying process, the obtained multiparticulates were dried in the fluid bed at temperatures up to 65°C.

Additional layer containing the surfactant (SDS) can be added as described in example 12.

**Table 1**

| Protection layered pellets, 5mg everolimus | | | | | |
|---|---|---|---|---|---|
| | | With 15% weight gain protection layer | | With 10% weight gain protection layer | |
| Processing step | Ingredients | % | mg/unit | % | mg/unit |
| Active layering | Sugar spheres 355-425 µm | 76.64 | 305.29 | 82.6 | 277.52 |
| | Everolimus | 1.30 | 5.00 | 1.50 | 5.00 |
| | Butyl Hydroxy Toluol | 0.03 | 0.10 | 0.03 | 0.10 |
| | Hypromellose 2910 3 cP | 5.22 | 20.00 | 6.0 | 20.00 |
| | Titan Dioxide | 0.22 | 0.84 | 0.3 | 0.84 |
| | Talc | 0.55 | 2.10 | 0.6 | 2.10 |
| Protectio n layer coating | Hypromellose 2910 3 cP | 10.03 | 38.46 | 7.0 | 23.51 |
| | Talc | 2.51 | 9.62 | 1.7 | 5.88 |
| | Titan Dioxide | 0.50 | 1.92 | 0.3 | 1.18 |
| Total: | | 100.00 | 383.33 | 100.00 | 336.12 |

### Example 2:

### Protection layered pellets for a dose of 20mg everolimus:

In this example another variant of pellets produced by layering and coating is provided. Immediate release pellets have higher drug load than in example 1 allowing for the manufacture of higher dose strengths. With this variant 10 or 20 mg can be filled into hard capsule of size 1. The material can be used of different kind of extended release coatings. Multiparticulates layered with a matrix containing the active and subsequently layered with a protective layer are produced as described in example 1. Deviant from example 1, the matrix forming polymer HPMC (type 2910, 3 cP) was dispersed in ethanol at a ratio of 3:2 related to the drug substance with a final concentration of 5% in the solvents. The concentration of active in the active layered pellets was increased form 1.5% in example 1 to 10%.

Additional layer containing the surfactant (SDS) can be added as described in example 12.

**Table 2:**

| Protection layered pellets, 20mg everolimus | | | |
|---|---|---|---|
| Processing step | Ingredients | % | mg/unit |
| Active layering | Sugar spheres 355-425 µm | 66.22 | 145.67 |
| | Everolimus | 9.09 | 20.00 |
| | Butyl Hydroxy Toluol | 0.18 | 0.40 |
| | Hypromellose 2910 3 cP | 13.57 | 29.85 |
| | Talc | 1.85 | 4.07 |
| Protection layer coating | Hypromellose 2910 3 cP | 6.99 | 15.38 |
| | Talc | 1.75 | 3.85 |
| | Titan Dioxide | 0.35 | 0.77 |
| Total: | | 100.00 | 220.00 |

### Example 3:

### Extended release pellets 5mg coated with Eudragit RS/RL

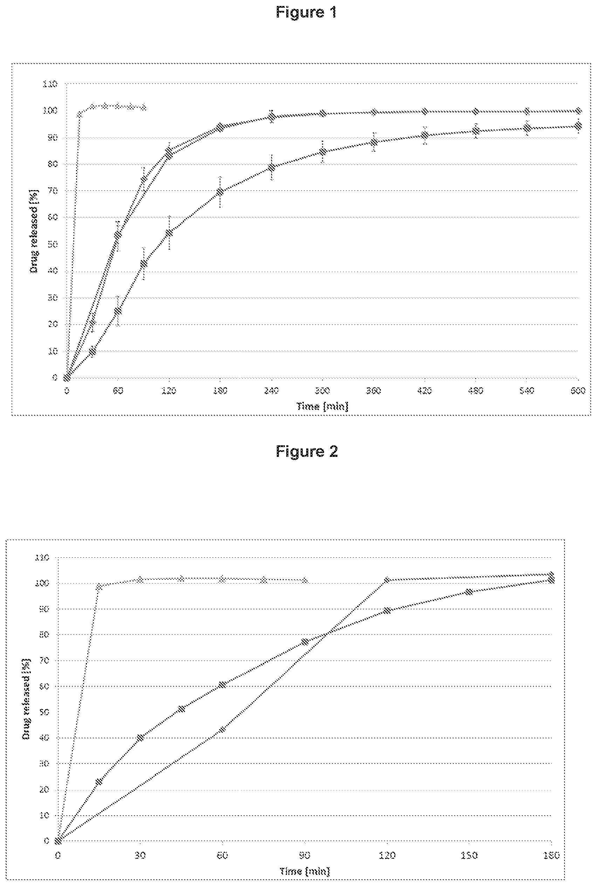

This example is providing a possibility how an extended release profile can be achieved by top coating of immediately release pellets such as pellets from table 3. A combination of insoluble polymers Eudragit RS and Eudragit RL can be properly adjusted to lead to a product with the desired release properties. In this case release was completed within 2 hours (see figure 2).
A coating was applied to the protective layered multiparticulates to obtain a product with sustained release properties:
Sustained release polymers Eudragit RL100 and Eudragit RS100 at a ratio of 3:7 were dissolved in acetone obtaining a final concentration of 14% in the solvents. While the solution was continuously stirred, 5% anti-tack agent glyceryl monostearate and 10% plasticizer triethylcitrate were added and dissolved at an amount relative to the amount of ammonio methacrylic acid copolymer (Eudragit RS/RL). A small fraction of water equal to 5% of total amount of solvents was used for dispersing 30% talc with the aid of a homogenizer. The aqueous suspension was added to the polymer solution.
The protective layered multiparticulates were preheated and fluidized in a fluid bed processor prior to starting spraying the dispersion. The spraying was conducted at a controlled product bed temperature in the range between 35° and 45°C using a bottom spray process until a polymer weight gain of 14% was received. After finishing the spraying process, the obtained multiparticulates were dried in the fluid bed at temperatures at 40°C for 15 min.
Finally the coated multiparticulates were filled manually into HPMC hard capsules of size 0. The fill weight was adjusted to amount equivalent to 5 mg everolimus.

Additional layer containing the surfactant (SDS) can be added as described in example 12.

**Table 3**

| Pro-cessing step | Ingredients | % | mg/unit |
|---|---|---|---|
| Active layering | Sugar spheres 355-425 µm | 83.3 | 305.29 |
| | Everolimus | 1.4 | 5.00 |
| | Butyl Hydroxy Toluol | 0.03 | 0.10 |
| | Hypromellose 2910 3 cP | 5.5 | 20.00 |
| | Titan Dioxide | 0.2 | 0.84 |
| | Talc | 0.6 | 2.10 |
| Protection layer coating | Hypromellose 2910 3 cP | 7.0 | 25.64 |
| | Talc | 1.7 | 6.41 |
| | Titan Dioxide | 0.3 | 1.28 |
| Total: | | 100.0 | 366.67 |

**Table 4:**

| Extended release coated multiparticulates Everolimus 5mg, Eudragit RS/RL 7:3, 16.9% polymer weight increase | | | |
|---|---|---|---|
| Processing step | Ingredients | % | mg/unit |
| Top coating | Protection layered pellets 5mg, Table1 | 80.2 | 366.67 |
| | Eudragit RS 100 | 9.5 | 43.00 |
| | Eudragit RL 100 | 4.1 | 19.00 |
| | Talc | 4.1 | 18.9 |
| | Glyceryl Monostearate | 0.7 | 3.15 |
| | Triethyl citrate | 1.4 | 6.30 |
| Total: | | 100.00 | 440.80 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3., respectively.

### In-vitro dissolution results:

The release profile is shown in figure 2.

| Minutes | % released Table 4 |
|---|---|
| 60 | 43.2 |
| 120 | 101.3 |
| 180 | 103.5 |

### Example 4:

### Extended release pellets 5mg coated with Eudragit RURS

A very fast releasing coating was applied to protection layered pellets with a drug load of 2.6% using only Eudragit RL100 as polymer. The coating spray fluid was prepared using a solvent mixture of isopropanol/acetone 60:40. The polymer concentration in the solvent was set to 10%(w/w). The polymer weight increase was 7.4%.

Additional layer containing the surfactant (SDS) can be added as described in example 12.

**Table 5**

| Protection layered pellets with 2.6% drug load Everolimus 5mg | | | |
|---|---|---|---|
| Processing step | Ingredients | % | mg/unit |
| Active layering | Sugar spheres 355-425 µm | 64.6 | 138.62 |
| | Everolimus | 2.3 | 5.00 |
| | Butyl Hydroxy Toluol | 0.05 | 0.10 |
| | Hypromellose 2910 3 cP | 9.3 | 20.00 |
| | Titanium Dioxide | 0.4 | 0.84 |
| | Talc | 1.0 | 2.10 |
| Protection layer coating | Hypromellose 2910 3 cP | 9.0 | 19.23 |
| | Talc | 2.2 | 4.81 |
| | Titan Dioxide | 0.4 | 0.96 |
| Total: | | 100.00 | 191.67 |

**Table 6:**

| Extended release coated multiparticulates Everolimus 5mg, Eudragit RS/RL 1:1, 7.4% polymer weight increase | | | |
|---|---|---|---|
| Processing step | Ingredients | % | mg/unit |
| Top coating | Protection layered pellets 5mg, Table 5 | 89.3 | 191.67 |
| | Eudragit RL 100 | 3.3 | 7.19 |
| | Eudragit RS 100 | 3.3 | 7.19 |
| | Talc | 3.3 | 7.19 |
| | Triethyl citrate | 0.7 | 1.44 |
| Total: | | 100.00 | 214.68 |

### Example 5:

### Extended release pellets for 5mg with use of pore former HPC:

The targeted release profile can be gained by applying a top coating onto protection layered pellets, which contains a certain fraction of pore forming agent. In this example the water soluble polymer hydroxypropyl cellulose was used to form pores in an insoluble ethylcellulose coating. Pellets layered with a matrix containing the active and subsequently layered with a protective layer were produced as described in example 1.
A coating was applied to the protective layered multiparticulates to obtain a product with sustained release properties.
10% lubricant colloidal dioxide and 10% plasticizer triethyl citrate based on amount of polymer were dispersed in ethanol. Then, sustained release polymer ethyl cellulose N-10 (EC) was dissolved with a final concentration of 6 to 7.5% in the solvents. While the dispersion was continuously stirred, HPC (Klucel EF) was added and dissolved at an amount equal to 45% to 50% of the amount of ethyl cellulose.
The protective layered multiparticulates were preheated and fluidized in a fluid bed processor prior to starting spraying the dispersion. The spraying was conducted at a controlled product bed temperature in the range between 35° and 45°C using a bottom spray process until a polymer weight gain of 7.5% to 11% was received. After finishing the spraying process, the obtained multiparticulates were dried in the fluid bed at temperatures up to 55°C.
Finally the coated multiparticulates were filled on an automatic capsule filling machine equipped with a dosing chamber filling station into HPMC hard capsules of size 0. The fill weight was adjusted to amount equivalent to 5 mg everolimus.

Additional layer containing the surfactant (SDS) can be added as described in example 12.

**Table 7:**

| Extended release coated multiparticulates (EC to pore former HPC ratio: 100:38, 10% weight gain Ethylcellulose) Everolimus 5mg | | | |
|---|---|---|---|
| Processing step | Ingredients | % | mg/unit |
| Top coating | Protection layered pellets 5mg, table 1 | 86.36 | 383.33 |
| | Ethylcellulose N-10 | 8.63 | 38.33 |
| | Hydroxypropylcellulose 300-600 cP | 3.28 | 14.57 |
| | Aerosil 200 | 0.86 | 3.83 |
| | Triethyl citrate | 0.86 | 3.83 |
| Total: | | 100.00 | 443.90 |

**Table 8:**

| Extended release coated multiparticulates (45% pore former HPC, 7.5% weight gain Ethylcellulose) Everolimus 5mg | | | |
|---|---|---|---|
| Processing step | Ingredients | % | mg/unit |
| Top coating | Protection layered pellets 5mg, table 1 | 89.0 | 383.33 |
| | Ethylcellulose N-10 | 6.7 | 28.75 |
| | Hydroxypropylcellulose 300-600 cP | 3.0 | 12.94 |
| | Aerosil 200 | 0.7 | 2.88 |
| | Triethyl citrate | 0.7 | 2.88 |
| Total: | | 100.00 | 430.77 |

**Table 9:**

| Extended release coated multiparticulates (EC to pore former HPC ratio: 100:50, 10.8% weight gain Ethylcellulose) Everolimus 5mg | | | |
|---|---|---|---|
| Processing step | Ingredients | % | mg/unit |
| Top coating | Protection layered pellets 5mg, table 1 | 84.2 | 336.12 |
| | Ethylcellulose N-10 | 9.2 | 36.67 |
| | Hydroxypropylcellulose 300-600 cP | 4.6 | 18.33 |
| | Aerosil 200 | 0.9 | 3.67 |
| | Triethyl citrate | 0.9 | 3.67 |
| Total: | | 100.00 | 398.46 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3. respectively.

### In-vitro dissolution results:

The release profile is shown in figure 1.

| Minutes | % released table 7 | % released table 8 | % released table 9 |
|---|---|---|---|
| 30 | 9.85 | 20.7 | -- |
| 60 | 24.9 | 53.0 | 53.8 |
| 120 | 54.4 | 85.32 | 83.3 |
| 180 | 69.6 | 94.3 | 93.5 |
| 240 | 78.8 | 97.7 | 97.9 |
| 300 | 84.7 | 99.0 | 99.1 |

### Example 6:

### Sustained release pellets for 5mg with use of pore-former HPMC:

Alternatively to example 5, other soluble polymers are also suitable to form pores in insoluble coatings in order to allow for a release of the drug form the pellets. Hypromellose (HPMC) can be used instead of HPC resulting in altered release profile. In this case almost 90% of drug can be released within 2 hours.
Pellets layered with a matrix containing the active and subsequently layered with a protective layer were produced as described in example.
A coating was applied to the protective layered multiparticulates to obtain a product with sustained release properties:
10% lubricant colloidal dioxide and 10% plasticizer triethyl citrate based on amount of polymer were dispersed in ethanol. Then, sustained release polymer ethyl cellulose N-10 was dissolved with a final concentration of 6 to 7.5% in the solvents. While the dispersion was continuously stirred, HPC (Klucel EF) was added and dissolved at an amount equal to 45% to 50% of the amount of ethyl cellulose.
The protective layered multiparticulates were preheated and fluidized in a fluid bed processor prior to starting spraying the dispersion. The spraying was conducted at a controlled product bed temperature in the range between 35° and 45°C using a bottom spray process until a polymer weight gain of 7.5% to 11% was received. After finishing the spraying process, the obtained multiparticulates were dried in the fluid bed at temperatures up to 55°C.
Finally the coated multiparticulates were filled on an automatic capsule filling machine equipped with a dosing chamber filling station into HPMC hard capsules of size 0. The fill weight was adjusted to amount equivalent to 5 mg everolimus.

Additional layer containing the surfactant (SDS) can be added as described in example 12.

**Table 10:**

| Sustained and delayed release coated pellets (EC to pore former HPMC ratio: 100:50, 5% weight gain Ethylcellulose) Everolimus 5mg | | | |
|---|---|---|---|
| Processing step | Ingredients | % | mg/unit |
| Top coating | Protection layered pellets 5mg, Table 4 | 91.7 | 191.67 |
| | Ethylcellulose N-10 | 4.6 | 9.58 |
| | HPMC 2910 3 cP | 2.3 | 4.79 |
| | Aerosil 200 | 0.7 | 1.44 |
| | Triethyl citrate | 0.7 | 1.44 |
| Total: | | 100.00 | 208.92 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3., respectively.

### In-vitro dissolution results:

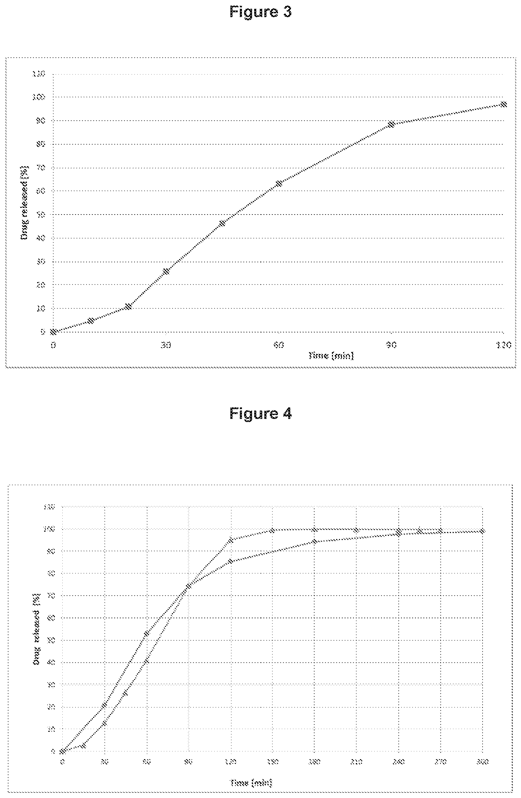

The in-vitro dissolution method as described in example 5 was used.
The release profile is shown in figure 3.

| Minutes | % released table 10 |
|---|---|
| 30 | 23.0 |
| 60 | 60.7 |
| 120 | 89.4 |
| 180 | 96.7 |

### Example 7:

### Sustained release minitablets coated with Eudragit RL/RS:

This example describes a possibility to use minitablets instead of pellets as substrate for extended release coating. A combination of permeable, insoluble polymer Eudragit RS with Eudragit RL was used to achieve retarded release.

A solid dispersion was manufactured with a solvent evaporation process. Solid dispersion consisted of everolimus and HPMC 2910 3 cp at ratio of 1:9 parts, and in addition lactose and BHT. The amount of BHT was 2% related to the amount of everolimus.

Everolimus was dissolved in a solvent mixture of ethanol and acetone at a ratio of 1:1, and then subsequently BHT, HPMC and Lactose added to the vessel and suspended. The dispersion was dried in vacuum with drier wall temperature of 50°C.

**Table 11:**

| Everolimus Solid Dispersion 9.09% | | | |
|---|---|---|---|
| Processing step | Ingredients | % | mg/unit |
| Solid dispersion | Everolimus | 9.09 | 5.00 |
| | BHT | 0.18 | 0.10 |
| | Lactose anhydrous | 8.91 | 4.90 |
| | HPMC 29120 3 cP | 81.82 | 45.01 |
| Total: | | 100.00 | 55.01 |

For the manufacture of the minitablets everolimus solid dispersion 9.09%, lactose anhydrous, microcrystalline cellulose and magnesium stearate were mixed with a turbula mixer for 5 minutes. The blend was compressed on a single punch tabletting machine using a minitablet punch tool with 19 punches of 2 mm in diameter. A compression force of approximately 18 kN was applied obtaining minitablets with sufficient tablet hardness of more than 10 N (range: 14-25 N) allowing for coating process.

**Table 12:**

| Minitablets 5mg everolimus | | | |
|---|---|---|---|
| Processing step | Ingredients | % | mg/unit |
| Solid dispersion | Everolimus Solid Dispersion 9.09%, table 11 | 27.5 | 55.01 |
| Tabletting | Lactose anhydrous | 41.5 | 82.99 |
| | Microcrystalline cellulose | 30.0 | 60.00 |
| | Magnesium stearate | 1.0 | 5.00 |
| Total: | | 100.00 | 200.00 |

The minitablets were coated on the lab scale fluid bed coater. A solution of Eudragit RL100 and Eudragit RS100 in a solvent mixture of isopropanol/acetone/water in a ratio of 55.8 : 37.2 : 7.0 was prepared. Plasticizer triethyl citrate and anti-tacking agent talc were added. The minitablets were fluidized in the processor with inlet air heated to 27-28°C and coated with a bottom spray process applying a spray pressure of 0.8 Bar.

**Table 13:**

| Sustained release coated minitablets, 5mg everolimus | | | |
|---|---|---|---|
| Processing step | Ingredients | % | mg/unit |
| Top coating | Minitablets everolimus 5mg, table 12 | 89.3 | 191.67 |
| | Eudragit RL 100 | 4.5 | 9.59 |
| | Eudragit RS 100 | 2.2 | 4.79 |
| | Talc | 3.3 | 7.19 |
| | Triethyl citrate | 0.7 | 1.44 |
| Total: | | 100.00 | 214.67 |

The immediate release formulation can be added.

### Example 8:

### Sustained release minitablets coated with Ethylcellulose and pore former HPC:

In this example a coating with pore formers was sprayed onto minitablets.
Minitablets were manufacture as described for example 7.
A lab scale fluid bed coater was used for a bottom spray coating process. In absolute ethanol the plasticizer triethyl citrate and anti-tacking agent colloidal silicon dioxide were dispersed before the coating polymer ethylcellulose N10 and the pore former HPC EF was dissolved. The minitablets were fluidized in the processor with inlet air heated to 43-45°C and coated with spray pressure of 0.8 bar.

Additional layer containing the surfactant (SDS) can be added as described in example 12.

**Table 14:**

| Sustained release coated minitablets, EC to pore former HPC ratio 1:1, 7.5% weight gain for Ethylcellulose Everolimus 5mg | | | |
|---|---|---|---|
| Processing step | Ingredients | % | mg/unit |
| Coating | Minitablets everolimus 5mg, table 12 | 82.64 | 200.00 |
| | Ethylcellulose N10 | 6.20 | 15.00 |
| | HPC EF | 6.20 | 15.00 |
| | Triethylcitrate | 1.24 | 3.00 |
| | Aerosil 200 | 3.72 | 9.00 |
| Total: | | 100.00 | 242.00 |

### In-vitro dissolution results:

The in-vitro dissolution method as described in example 5 was used.

The release profile is shown in figure 3.

| Minutes | % released table 14 |
|---|---|
| 30 | 25.8 |
| 60 | 63.2 |
| 90 | 88.4 |
| 120 | 97.0 |

### Example 9:

### 20mg capsule filled with sustained release coated pellets using coating polymer ethylcellulose and pore former HPC:

In this example pellets with higher drug load were used for the filling of hard capsule of size 1 with dose strength of 10 or 20 mg. The product could be improved with respect to its chemical stability by the use of HPMC capsules and the addition of the superdisintegrant crospovidone with high moisture binding capacity.
Pellets layered with a matrix containing the active, and subsequently layered with a protective layer, were produced as described in example 2.
A coating was applied to the protective layered multiparticulates according to process described in example 5. The polymer concentration (EC and HPC) in the spray fluid were set to 10%. The amount of plasticizer HPC and anti-tacking agent Aerosil were used at an amount of 10% relative to polymers EC and HPC.

The pellets were filled into HPMC capsule of size 1, and subsequently crospovidone was filled in the same process at a second filling station separately into the capsules.

Additional layer containing the surfactant (SDS) can be added as described in example 12.

**Table 15:**

| Extended release coated pellets in capsules (EC to pore former HPC ratio: 100:42, 7.5% weight gain Ethylcellulose) 20mg everolimus | | | |
|---|---|---|---|
| Processing step | Ingredients | % | mg/unit |
| Top coating | Protection layered pellets 20mg, table 2 | 88.7 | 220.00 |
| | Ethylcellulose N-10 | 6.7 | 16.50 |
| | Hydroxypropylcellulose 300-600 cP | 0.9 | 6.93 |
| | Aerosil 200 | 0.9 | 2.34 |
| | Triethyl citrate | 2.8 | 2.34 |
| Capsule filling | Crospovidone | n.a | 50.00 |
| | Capsule shell Qualicaps V (HPMC) size 1 | n.a. | 70.00 |
| Total: | | 100.00 | 368.12 |

### Example 10:

This example demonstrates that it is feasible to use extended release coated pellets as described in the examples above for a tabletting process in order to obtain a tablets as alternative dosage form.

Extended release coated pellets as used in example 9 for filling of hard capsules were alternatively mixed with filler microcrystalline cellulose, glidant colloidal silicon dioxide and lubricant magnesium stearate in a tumble bin blender to obtain a suitable blend for tabletting. The pellet concentration in the blend was kept at 40% in order to gain a mechanically stable tablet with fully embedded coated pellets. On a single punch machine round, biconvex tablets of 9 mm diameter were compressed with a compression force of 4 kN obtaining a tablet hardness of 38 N. The tablets disintegrated fast and the drug release of the tableted pellets was only marginally impacted by the compaction as it can be seen by dissolution results.

The same can be done with pellets containing a surfactant layer or a layer with high drug load.

**Table 16:**

| Extended release coated pellets in capsules (EC to pore former HPC ratio: 100:42, 7.5% weight gain Ethylcellulose) 10mg everolimus | | | |
|---|---|---|---|
| Processing step | Ingredients | % | mg/unit |
| Tabletting | Extended Release pellets 10mg Example 9 / table 15 | 40.00% | 107.92 |
| | Microcrystalline Cellulose PH200 | 14.50 | 39.12 |
| | Microcrystalline Cellulose PH102 | 43.50 | 117.36 |
| | Aerosil 200 | 1.00 | 2.70 |
| | Magnesium Stearate | 1.00 | 2.70 |
| Total: | | 100.00 | 269.80 |

### In-vitro dissolution results:

The in-vitro dissolution method as described in example 5 was used.

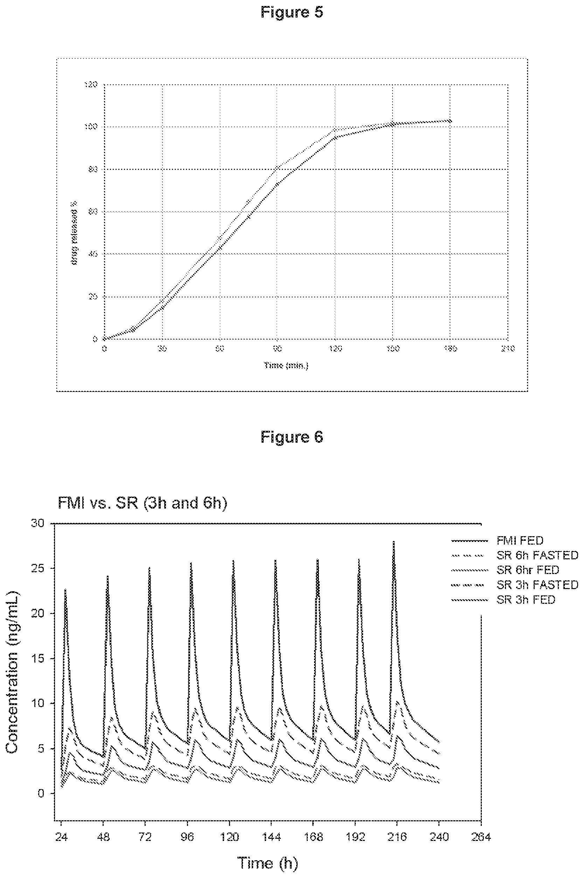

The release profile is shown in figure 6.

| Minutes | Pellets % released table 15 | 10mg tablet % released table 16 |
|---|---|---|
| 30 | 14.8 | 17.9 |
| 60 | 42.9 | 47.4 |
| 120 | 94.9 | 98.4 |
| 180 | 102.9 | 102.5 |

### Example 11:

Extended release profiles can be also achieved by forming diffusion controlled matrix system instead of applying a coating. In this example an extended release matrix is presented where two grades of hypromellose (HPMC) with different viscosities were combined to obtain a swellable, high viscous matrix system with specific release profile.

All amounts of excipients were weighed, sieved and filled into the container of blender, e.g. tumble bin mixer, and were mixed for a suitable time. Magnesium stearate was added not before 5 minutes of the suitable blending time was left to ensure good lubrication during tabletting. The blend was compressed on a single punch tabletting machine using a minitablet punch tool with 19 punches of 2 mm in diameter. A compression force of approximately 12 kN was applied obtaining minitablets with sufficient tablet hardness of more than 15 N.

**Table 17:**

| Extended release matrix minitablets 5 mg everolimus | | | |
|---|---|---|---|
| Processing step | Ingredients | % | mg/unit |
| Blending and Tabletting | Everolimus solid dispersion 9.09% | 22.92 | 55.01 |
| | Methocel K100 LVP CR | 37.50 | 90.00 |
| | Pharmacoat 603 | 12.50 | 30.00 |
| | Lactose anhydrous | 24.58 | 58.99 |
| | Crospovidone XL10 | 1.00 | 2.40 |
| | Colloidal Silicon Dioxide | 0.50 | 1.20 |
| | Magnesium stearate | 1.00 | 2.40 |
| Total: | | 100.00 | 240.00 |

Table 18 (next page): pharmacokinetic parameters from human study comparing 3 different formulations at a single dose of 10mg in fed and fasted state:
IR: conventional, immediate release, fast disintegrating tablet
SR 3h: sustained release pellets in a HPMC capsule size 0, 5mg everolimus per capsule, approx. 90% everolimus released in 3h, example 5 / table 8
SR 6h: sustained release pellets in a HPMC capsule size 0, 5mg everolimus per capsule approx. 90% everolimus released in 6h, example 5 / table 7

| | | IR | SR 3h FED | SR 3h FAST | SR 6h FED | SR 6h FAST |
|---|---|---|---|---|---|---|
| t_{1/2} | Mean | 36.7 | 38.5 | 37.4 | 37.9 | 46.9 |
| | SD | 6.20 | 7.81 | 11.40 | 13.7 | 21.7 |
| | CV% | 16.9 | 20.3 | 30.5 | 36.1 | 46.2 |
| tₘₐₓ | Mean | 1.81 | 4.58 | 4.29 | 4.61 | 4.83 |
| | SD | 0.66 | 1.08 | 1.14 | 1.73 | 1.46 |
| | CV% | 36.3 | 23.6 | 26.5 | 37.5 | 30.2 |
| | Range | (1-3) | (3-6) | (2.5-6) | (2.5-8) | (2.5-6) |
| Cₘₐₓ | Mean | 30.16 | 3.61 | 4.29 | 1.91 | 1.76 |
| | SD | 9.58 | 0.946 | 1.14 | 0.488 | 0.452 |
| | CV% | 31.7 | 26.2 | 26.5 | 25.5 | 25.7 |
| C₂₄ₕ | Mean | 2.79 | 1.31 | 1.80 | 0.68 | 0.82 |
| | SD | 0.670 | 0.371 | 0.416 | 0.150 | 0.220 |
| | CV% | 24.0 | 28.3 | 23.2 | 22.1 | 26.8 |
| Cₘₐₓ/C₂₄ₕ | | 10.8 | 2.74 | 2.38 | 2.8 | 2.14 |
| New formulation/FMI % | | | 46.9 | 64.4 | 24.4 | 29.4 |
| AUC_{inf} | Mean | 285.8 | 117.1 | 160.7 | 61.2 | 80.0 |
| | SD | 66.5 | 28.5 | 44.1 | 12.5 | 20.9 |
| | CV% | 23.3 | 24.4 | 27.4 | 20.5 | 26.1 |
| Bioavailability | | | 41.0 | 56.2 | 21.4 | 28.0 |
| Fed vs. Fasted | | | 72.9 | | 76.5 | |

### Example 12

This example describes how the release rate of an extended release formulation as described examples 1 to 11 can be modulated by spraying an additional layer containing the surfactant (for example sodium dodecyl sulphate, SDS) on the inert starter core, followed by a drug substance containing layer, a protection layer and an extended release coating, optionally containing water soluble excipients. The additional layer comprising a surfactant is located beneath the active substance containing layer. This separation avoids a direct contact of the surfactants with the active drug substance. The desired release properties can be fine-tuned by combined action of the surfactant layer, drug substance containing layer and the top coating with extended release properties. The latter can further contain water soluble excipients to further modulate the active ingredient release rate. For one example with the applied process the active ingredient release was significantly increased in presence of the surfactant containing layer compared to a similar surfactant free system (see table 19 for in-vitro dissolution data).

### Step1:

A procedure for preparing multiparticulates with a drug containing matrix layer was as follows: For manufacture of the surfactant containing layer, the matrix forming polymer HPMC (type 2910, 3 cP) was dispersed in ethanol with a final concentration of about 94% in the solvent. A small fraction of water equal to about 6% of total amount of solvents was used for dispersing the talc in the layer and to dissolve sodium dodecyl sulphate (SDS, surfactant; also poloxamer 188 or TPGS could be used). The aqueous suspension was added to the dispersion. During continuous stirring the dispersion was equilibrated until the swollen polymer particles were disintegrated. Finally, the feed was layered onto sugar spheres of 250 to 355 µm size in a fluid bed processor (for exact feed composition see Table 1). The spraying occurred at a controlled product bed temperature in the range between 34° and 45°C, preferably between 34°C to 38°C, or between 35°C to 37°C using a tangential spray process. The obtained multiparticulates were dried in the fluid bed at temperatures up to 55°C.

### Step 2:

The matrix forming polymer HPMC (type 2910, 3 cP) was dispersed in ethanol at a ratio of 4:1 related to Everolimus with a final concentration of 6% in the solvents. Antioxidant butyl hydroxy toluol was added to the dispersion at an amount as indicated in Table 2. A small fraction of water equal to 6% of total amount of solvents was used for dispersing the talc. The aqueous suspension was added to the dispersion. During continuous stirring the dispersion is equilibrated until the swollen polymer particles were disintegrated. Finally, the drug substance was added and dispersed in the coating dispersion prior to starting the layering onto the sugar spheres of 250 to 355 µm derived from step 1, preheated and fluidized in a fluid bed processor. The amount of sugar spheres used resulted in a drug concentration of about 18% in the active layer after spraying. The spraying occurred at a controlled product bed temperature in the range between 35° and 45°C using a tangential spray process. The obtained multiparticulates were dried in the fluid bed at temperatures up to 55°C.

### Step 3:

A subsequent layering procedure followed for applying a protective, stability enhancing layer: The binding polymer HPMC (type 2910, 3 cP) was dispersed in ethanol with a final concentration of 7% in the solvents. A small fraction of water equal to 6% of total amount of solvents was used for dispersing talc and titanium dioxide with the aid of a homogenizer. The aqueous suspension was added to the dispersion. During continuous stirring the dispersion was equilibrated until the swollen polymer particles were disintegrated. The active layered multiparticulates were preheated and fluidized in a fluid bed processor. The spraying was conducted at a controlled product bed temperature in the range between 35° and 45°C using a bottom spray process. After finishing the spraying process, the obtained multiparticulates were dried in the fluid bed at temperatures up to 55°C.

### Step 4:

The protective layered multiparticulates were preheated and fluidized in a fluid bed processor prior to starting spraying the dispersion. The two polymers, Triethylcitrate and Aerosil 200 were added dissolved and dispersed in Ethanol with a final concentration of 11% in the solvent.

The spraying was conducted at a controlled product bed temperature in the range between 35° and 45°C using a bottom spray process until a polymer weight gain of 14% was received. After finishing the spraying process, the obtained multiparticulates were dried in the fluid bed at temperatures at 40°C for 15 min.

Finally the coated multiparticulates were filled manually into HPMC hard capsules of size 0. The fill weight was adjusted to amount equivalent to 5 mg everolimus.

**Table 1**

| Pro-cessing step | Ingredients | % | mg/unit |
|---|---|---|---|
| Surfactant containing layer | Sugar spheres 250-355 µm | 80.8 | 203.55 |
| | SDS | 2.98 | 7.50 |
| | Hypromellose 2910 3 cP | 14.89 | 37.50 |
| | Talc | 1.34 | 3.38 |
| Total: | | 100.0 | 251.93 |

**Table 2**

| Pro-cessing step | Ingredients | % | mg/unit |
|---|---|---|---|
| Active layering | Pellets with surfactant layer of Table 1 | 75.6 | 251.93 |
| | Everolimus | 4.50 | 15.00 |
| | Butyl Hydroxy Toluol | 0.09 | 0.30 |
| | Hypromellose 2910 3 cP | 18.00 | 60.00 |
| | Talc | 1.83 | 6.11 |
| Total: | | 100.0 | 333.33 |

**Table 3: Protection coating**

| Pro-cessing step | Ingredients | % | mg/unit |
|---|---|---|---|
| | Active layered pellets | 90.91 | 333.33 |
| Protection layer coating | Hypromellose 2910 3 cP | 6.99 | 25.64 |
| | Talc | 1.75 | 6.41 |
| | Titan Dioxide | 0.35 | 1.28 |
| Total: | | 100.0 | 366.67 |

**Table 4: Top coating**

| Extended release coated multiparticulates | | | |
|---|---|---|---|
| Everolimus 5mg, Eudragit RS/RL 7:3, 16.9% polymer weight increase | | | |
| Processing step | Ingredients | % | mg/unit |
| Top coating | Protection layered pellets | 88.67 | 366.67 |
| | Ethylcellulose N-10 | 6.65 | 27.50 |
| | Hydroxypropylcellulose 300-600 cP | 2.79 | 11.55 |
| | Aerosil 200 | 0.95 | 3.91 |
| | Triethyl citrate | 0.95 | 3.91 |
| Total: | | 100.00 | 413.54 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3., respectively.

**Table 19: in-vitro dissolution results:**

| Minutes | % released Formulation without SDS layer | % released Formulation with SDS layer |
|---|---|---|
| | Table 2, Table 3 and Table 4 | Table 1, Table 2, Table 3 and Table 4 |
| 15 | 4.2 | 29.7 |
| 30 | 16.8 | 68.1 |
| 60 | 63.8 | 91.4 |
| 75 | 78.5 | 94.4 |
| 90 | 86.3 | 95.7 |
| 120 | 93.2 | 96.1 |
| 150 | 95.5 | 96.2 |
| 180 | 96.4 | 95.6 |

The dissolution can be further improved by adding immediate release part to the formulation.

The immediate release part, that can contain 40-O-(2-hydroxy)ethyl-rapamycin in a weight ratio relative to the rest of the 40-O-(2-hydroxy)ethyl-rapamycin in the formulation from 5:2 to 1:20, preferably is from 1:5 to 1:20; particularly is from 1:8 to 1:12, specifically is 1:10. The immediate release can fill the dissolution gap in the first minutes and thus leads to even more uniform release profile, which is further elucidated in example 13.

### Example 13

A combination of small amount of immediate release (IR) and the sustained release (SR) variant can improve the bioavailability of the combined formulation variant as illustrated by the simulated concentration-time profiles at steady-state below.

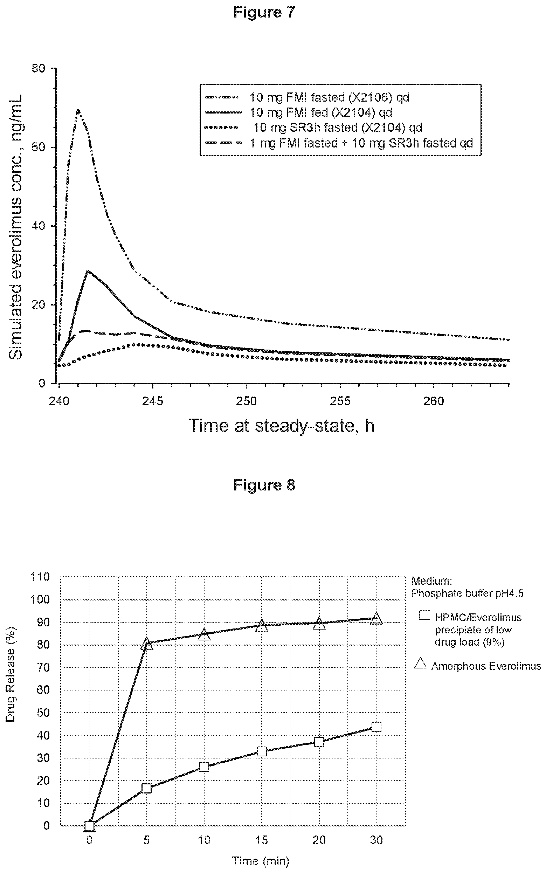

Conditions used in simulation: Simulated steady-state concentration-time profiles after daily administration of 10 mg FMI under fasting conditions, 10 mg FMI after a light fat meal, 10 mg SR-fast variant under fasting conditions, and a combination of 1 mg IR plus 10 mg SR-fast variant (IR+SR) under fasting conditions are shown in Figure 7. The concentration-time profiles at steady-state were simulated by using the nonparametric superposition function of the WinNonlin Version 6.2 based on the concentration data from Study X2104 and Study X2106:
- Simulated concentration-time profile after daily administration of 10 mg FMI under fasting conditions was simulated based on 2 x mean concentration data after a single 5 mg dose of FMI administered under fasting conditions in Study X2106
- Simulated concentration-time profile after daily administration of 10 mg FMI after a light fat meal was simulated based the mean concentration data after a single 10 mg dose of FMI administered after a light fat meal in Study X2104
- Simulated concentration-time profile after daily administration of 10 mg SR-fast variant under fasting conditions was simulated based the mean concentration data after a single 10 mg dose of the SR-fast variant administered under fasting conditions in Study X2104
- Simulated concentration-time profile after daily administration of 1 mg IR +10 mg SR-fast variant under fasting conditions was simulated by combining:
   ∘ 1/10 of the simulated concentration data at steady-state after 10 mg daily dose of the FMI administered under fasting conditions based on data in Study X2106 and
   ∘ Simulated concentration data at steady-state after 10 mg daily dose of SR-fast variant administered under fasting conditions based on data in Study X2104

Pharmacokinetic parameters of the simulated steady-state concentration-time profiles were estimated using WinNonlin Version 6.2 and are listed in Table 20.

**Table 20**

| | AUCtau (ng.h/mL) | Cmax (ng/mL) | Cmin (ng/mL) | Cmax/Cmin | Bioavailability (relative to 10 mg FMI fed) |
|---|---|---|---|---|---|
| 10 mg FMI fasted | 502 | 69.4 | 11.1 | 6.27 | 199% |
| 10 mg FMI fed | 252 | 28.6 | 6.00 | 4.76 | 100% |
| 10 mg SR-fast variant fasted | 157 | 9.92 | 4.57 | 2.17 | 62.2% |
| 1 mg IR + 10 mg SR-fast variant fasted | 207 | 13.4 | 5.68 | 2.35 | 74.7% |

| | | | | | |
|---|---|---|---|---|---|
| AUCtau = AUC during a dosing interval of 24 hours | | | | | |

The purpose of developing a sustained-release formulation for everolimus is to reduce the Cmax of the concentration-time profile while maintaining the same Cmin level as the current FMI formulation (i.e. formulation available on the market). Without wishing to be bound to any theory, this is based on the assumption that Cmax is related to toxicity and Cmin is related to efficacy. Thus, an ideal sustained-release formulation for everolimus is one with a reduced Cmax/Cmin ratio and with an appropriate dose to maintain Cmin similar to that after the 10 mg FMI qd.

The following can be summarized by the simulated steady-state concentration-time profiles in Figure 7:
- Light fat meal reduced AUC, Cmax, and the Cmax/Cmin ratio of the FMI formulation
- Daily 10-mg SR-fast variant had a lower Cmax/Cmin ratio (2.17) relative to that of the daily 10-mg FMI fed (4.76). However, the bioavailability of the daily 10-mg SR-fast variant was only 62.2% that of the daily 10-mg FMI fed. In addition, the daily 10 mg SR-fast variant also had a lower Cmin than the daily 10 mg FMI fed
- An addition of 1-mg IR to the 10-mg SR-fast variant (IR+SR) improves the bioavailability (from 62.2% to 74.7% relative to 10 mg FMI fed) and increases the steady-state Cmin of the SR-fast variant (from 4.57 to 5.68 ng/mL). The addition of 1-mg IR to the SR-fast variant increases the steady-state Cmax/Cmin ratio slightly from 2.17 to 2.35.
- Thus, the addition of 1-mg IR to 10-mg SR-fast variant can improve the performance of the SR-fast variant by:
   ∘ Improving the bioavailability
   ∘ Increasing the steady-state Cmin (for better efficacy)
   ∘ Maintaining similar steady-state Cmax/Cmin ratio (maintain the same better toxicity profile relative to 10 mg FMI fed)

### Example 14

The slow release formulation based on dissolution results obtained with pure amorphous everolimus, which was mixed with hydrophilic table excipients (dissolution shown in Figure 8 as detected in detergent free media). Amorphous everolimus in high drug load alone showed sustained release properties. Therefore, it is an option to achieve sustained release behaviour only with the active ingredient provided everolimus concentration in the layer is high enough to allow the physical-chemical surface properties of the everolimus to limit dissolution speed (Tables 21 and 22). The expected drug loading in the active layer in case the hydrophilic carrier polymer Hypromellose (e.g. 2910 3 cP) is used as a matrix/binder (Table 2A, Table 2B) is more than 40 %. More preferred the drug load can be between 45-90 wt%. An example of such a pellet system with a high drug load in the active layer and a protection layer on top of the active layer is shown as Example 15. In addition, the active layer can contain RAD001 in amorphous state and other antioxidants or matrix formers. Further excipients modulating the wettability of the system can optionally be added (surfactants, wetting agents). The everolimus' stability can further be safeguarded by specific binders, stabilizers or processing agents. The additives as described above for preparation of the formulation with the surfactant in a layer beneath the active ingredient layer can also be used.

### Example 15:

### Protection layered pellets for a dose of 20mg everolimus with high drug load in the drug substance containing layer:

In this example another variant of pellets produced by layering and coating is provided. With this variant 20 mg or even higher amounts can be filled into hard capsule of size 1. The material can be used of different kind of extended release coatings or without extended release top coating.
Multiparticulates layered with a matrix containing the active and subsequently layered with a protective layer were produced as described in example 1. Deviant from example 1, the matrix forming polymer HPMC (type 2910, 3 cP) was dispersed in ethanol at a ratio of 1:4 related to the drug substance (see table 21). The concentration of active in the active layered pellets was increased form 1.5% in example 1 to 10%.
A similar example with a 50% increased drug load in the active layer is given in Table 22. The concentration of the active ingredient in active layered pellets was in this case about 7%, compared to 1.5% as in example 1.

Additional layer containing the surfactant (e.g. SDS, TPGS or Poloxamer 188) can be added as described in example 12.

**Table 21:**

| Protection layered pellets, 20mg everolimus | | | |
|---|---|---|---|
| Processing step | Ingredients | % | mg/unit |
| Active layering | Sugar spheres 355-425 µm | 74.65 | 145.67 |
| | Everolimus | 10.25 | 20.00 |
| | Butyl Hydroxy Toluol | 0.20 | 0.40 |
| | Hypromellose 2910 3 cP | 2.57 | 5.00 |
| | Talc | 2.09 | 4.07 |
| Protection layer coating | Hypromellose 2910 3 cP | 7.88 | 15.38 |
| | Talc | 1.97 | 3.85 |
| | Titan Dioxide | 0.39 | 0.77 |
| Total: | | 100.00 | 195.15 |

**Table 22:**

| Active layered pellets, 15mg everolimus | | | |
|---|---|---|---|
| Processing step | Ingredients | % | mg/unit |
| Active layering | Sugar spheres 355-425 µm | 84.81 | 186.6 |
| | Everolimus | 6.82 | 15.00 |
| | Butyl Hydroxy Toluol | 0.18 | 0.40 |
| | Hypromellose 2910 3 cP | 6.82 | 15.00 |
| | Talc | 1.36 | 3.00 |
| Total: | | 100.00 | 220.00 |

### Example 16

Everolimus is completely compatible only with a very limited number of customary excipients. Table 23 shows compatibility results of Everolimus in a presence of different fillers and polymers as studied during development and confirms how paramount it can be to pharmaceutical formulation's stability to select appropriate excipients. Everolimus showed in general only a moderate compatibility to the filler and polymers which were tested (see table 23). It depicts how difficult it is to formulate a stable pharmaceutical formulation comprising everolimus.

### Stress test set-up:

Drug solution was prepared using a Everolimus to HPMC ration of 1:4 from ethanol and ethanol was evaporated to from a solid precipitate of 20% drug load. Selected excipients were either added into this organic suspension prior to drying or added to the dried HPMC/Everolimus precipitate for the compatibility studies. Rational for this two approaches was the expected distance of the excipient compared Everolimus in the envisaged formulation.

The mixtures were equilibrated to 20% relative humidity and subsequently stressed for 120 hours at 76.5°C.

**Table 23**

| Excipients tested in combination with 5% everolimus solid dispersion | % of everolimus remaining after the stress test |
|---|---|
| 1 Sugar spheres (Suglets 180-250 µm ground), Co-precipitated into the solid dispersion | 86.3 |
| Microcrystalline Cellulose spheres (Cellets 350, ground) Co- precipitated into the solid dispersion | 63.3 |
| Polyethylene Oxide 65-95 (Polyox WST N80) Co- precipitated into the solid dispersion | 40.5 |
| HPMC 2910 3 cP + 5% Titan Dioxide Co- precipitated in to the solid dispersion | 92.7 |
| Methocel K100 LVP + 5% Titan Dioxide | 86.8 |
| Co- precipitated in to the solid dispersion | |
| HPMC 2208 4000 cP +5% Titan Dioxide Co- precipitated in to the solid dispersion | 76.6 |
| Eudragit RL10 Co- precipitated in to the solid dispersion | 93.01 |
| HPMCAS-LF Added to the dry RAD/HPMC precipitate | 65.54 |
| Eudragit L100-55 Added to the dry RAD/HPMC precipitate | 0.00 (no everolimus left) |
| Ethylcellulose + 10% Triethyl citrate Added to the dry RAD/HPMC precipitate | 71.31 |
| HPC Added to the dry RAD/HPMC precipitate | 75.94 |

The enteric coating polymer Eudragit L100-55 is strongly incompatible with everolimus and no Everolimus was detected after the test despite the Eudragit L100-55 being only added as a dry powder to the dry HPMC precipitate containing everolimus (and not being directly precipitated into the active containing particles.

Everolimus is especially non-compatible to all acidic compounds like the enteric coating polymers HPMC-AS (HPMC AS-LH), and Eudragit L100. In addition it shows a significant incompatibility to Triethylcitrate mixtures which is normally used in coating layers as non-soluble polymer part, plasticizer or softener.

### Example 17

Everolimus with 0.2% Butylhydroxytoluene and the Hypromellose 2910 3 cP were dissolved in a mixture of ethanol:water (94:6) with a solid concentration of 9 wt%. Subsequently the solvents were evaporated under reduced pressure and elevated temperature (40°C) using a rotavapor-equipment. The resulting semi-solid material was transferred from the glass bulb of the rotavapor into an open glass beaker and drying was finished overnight in an vacuum oven under reduced pressure. Resulting solid material was crushed into a fine solid dispersion particles.

By applying this procedure solid dispersion with a Everolimus to Hypromellose ratio of 1:4 and of 4:1 was obtained.

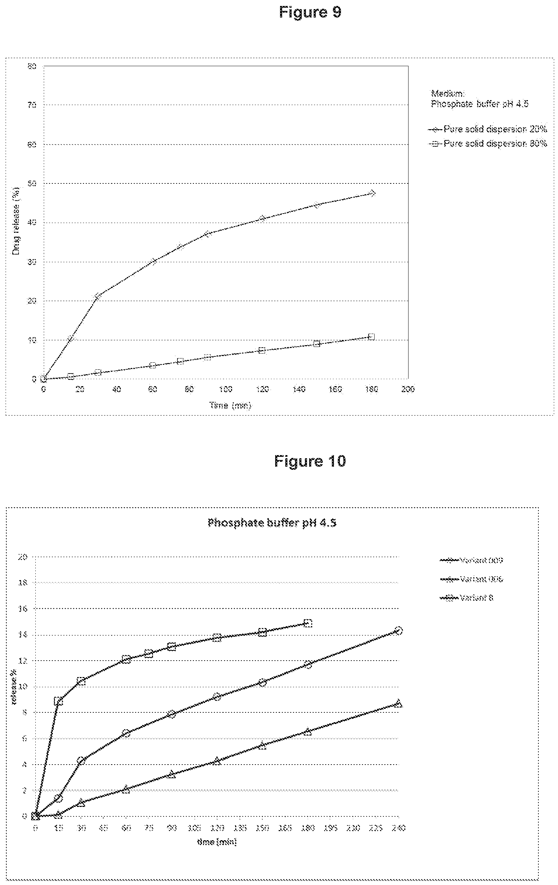

Dissolution which is seen by adding such solid dispersion particles with 10 mg everolimus into phosphate buffer medium at pH4.5 are shown in Figure 9. Whereas the solid dispersion of low drug load released under these test conditions about 50% of embedded Everolimus within 3 hours, similar solid dispersion particles with high drug load released only about 10% of the embedded drug.

### Example 18

### Extended release pellets with a high drug load (50%) and surfactant located in different layers

This example describes the influence of addition of surfactant in different layers on drug release. With this variants 20 mg or even higher amounts can be filled into hard capsule of size 1.

The Variants B, 006 and 009 are described in Table 24 to 27 and illustrated in Figure 10.

In Variant 009 the surfactant was located in the surfactant layer as described in example 12. In variant B the surfactant was located directly in the drug layer and variant 006 was without surfactant.

The extended release layer was sprayed with a composition comprising a water insoluble polymer (ethyl cellulose) and a pore former (hydroxy propyl cellulose) in a ratio of 100:70 for all three variants.

In Variant 009 the concentration of surfactant was 13.7 % based on the layer thickness and 17 % based on everolismus.

In Variant B the surfactant was added directly in the drug layer (see table 25) with a concentration of 10 % based on the layer thickness. The concentration based on everolismus was 24%. Variant 006 was sprayed without surfactant.

This example should illustrate the influence of surfactant on the drug release. The differences are very visible in phosphate buffer pH 4.5 due to the absence of surfactant in the medium.

### Preparation Surfactant layer:

The surfactant layer was applied on the inert starter core in order to enhance wettability of the drug substance and to stabilize the core prior to drug layering. The surfactant was dissolved in ethanol. Subsequently HPMC (type 2910, 3 cP) and talc were dispersed in the surfactant/ ethanol solution. A small fraction of water equal to 6% of total amount of solvents was used for dispersing titanium dioxide with the aid of a homogenizer. The aqueous suspension was added to the dispersion. During continuous stirring the dispersion was equilibrated until the swollen polymer particles were disintegrated. The starter cores were preheated and fluidized in a fluid bed processor. The spraying was conducted at a controlled product bed temperature in the range between 34° and 38°C using a bottom spray process. After finishing the spraying process, the obtained multiparticulates were dried in the fluid bed at temperatures up to 55°C.

### Preparation Drug layer:

The antioxidant butyl hydroxy toluol (2% based on drug substance) and the surfactant (if applicable) were dissolved in ethanol. The matrix forming polymer HPMC (type 2910, 3 cP) and talc were dispersed in ethanol. A small fraction of water (6%) of total amount of solvents was added to the dispersion. During continuous stirring the dispersion was equilibrated until the swollen polymer particles were disintegrated. Finally, the drug substance was added and dispersed in the coating dispersion prior to starting the layering onto the starter cores (sugar spheres or surfactant layered pellets), preheated and fluidized in a fluid bed processor. The concentration of drug substance was 41% (Variant B) to 46% (Variant 006, 009) based on the layer thickness. The spraying occurred at a controlled product bed temperature in the range between 34° and 38°C using a tangential spray process. The obtained multiparticulates were dried in the fluid bed at temperatures up to 55°C.

### Preparation Protective layer:

A subsequent layering procedure followed for applying a protective, stability enhancing layer: The binding polymer HPMC (type 2910, 3 cP) and talc were dispersed in ethanol. A small fraction of water equal to 6% of total amount of solvents was used for dispersing titanium dioxide with the aid of a homogenizer. The aqueous suspension was added to the dispersion. During continuous stirring the dispersion was equilibrated until the swollen polymer particles were disintegrated. The active layered multiparticulates were preheated and fluidized in a fluid bed processor. The spraying was conducted at a controlled product bed temperature in the range between 34° and 38°C using a bottom spray process. After finishing the spraying process, the obtained multiparticulates were dried in the fluid bed at temperatures up to 55°C.

### Preparation Extended release layer:

The extended release polymer ethyl cellulose and the pore former hydroxy propyl cellulose were added and dissolved in ethanol. Afterwards the plasticizer triethylcitrate and anticaking agent Aerosil 200 were added, dissolved and dispersed in Ethanol with a final concentration of 8% in the solvent.

The spraying was conducted at a controlled product bed temperature in the range between 34° and 38°C using a bottom spray process until a polymer weight gain of 20% was received. The fill weight was adjusted to amount equivalent to 5 mg and 20mg everolimus fitting in HPMC capsules size 1.

**Table 24 Surfactant layer**

| | | Variant 009 | | Variant 006 | | Variant B | |
|---|---|---|---|---|---|---|---|
| Processing step | Ingredients | % | mg/unit | % | mg/unit | % | mg/unit |
| Surfactant containing layer | Sugar spheres 250-355 µm | 83.33 | 30.16 | Not performed | | Not performed | |
| | SDS | 2.29 | 0.83 | | | | |
| | Hypromellose 2910 3 cP | 10.65 | 3.86 | | | | |
| | Talc | 3.20 | 1.16 | | | | |
| | Titan dioxide | 0.19 | 0.19 | | | | |
| Total: | | 100.0 | 36.20 | - | - | - | - |

**Table 25 Drug layer**

| | | Variant 009 | | Variant 006 | | Variant B | |
|---|---|---|---|---|---|---|---|
| Processing step | Ingredients | % | mg/unit | % | mg/unit | % | mg/unit |
| Drug layer | Sugar spheres 250-355 µm | - | - | 76.92 | 36.20 | 76.92 | 161.05 |
| | Surfactant layered pellets | 76.92 | 36.20 | - | - | - | - |
| | Everolimus | 10.63 | 5.00 | 10.63 | 5.00 | 9.55 | 20.00 |
| | BHT | 0.23 | 0.11 | 0.23 | 0.11 | 0.23 | 0.48 |
| | SDS | - | - | - | - | 2.31 | 4.83 |
| | Hypromellose 2910 3 cP | 10.63 | 5.00 | 10.63 | 5.00 | 9.55 | 20.00 |
| | Talc | 1.59 | 0.75 | 1.59 | 0.75 | 1.43 | 3.00 |
| Total: | | 100.00 | 47.05 | 100.00 | 47.05 | 100.00 | 209.37 |

**Table 26 Protective layer**

| | | Variant 009 | | Variant 006 | | Variant B | |
|---|---|---|---|---|---|---|---|
| Processing step | Ingredients | % | mg/unit | % | mg/unit | % | mg/unit |
| protective layer | Drug layered pellets | 83.33 | 47.05 | 83.33 | 47.05 | 83.33 | 209.37 |
| | Hypromellose 2910 3 cP | 12.35 | 6.97 | 12.35 | 6.97 | 12.35 | 31.02 |
| | Talc | 3.70 | 2.09 | 3.70 | 2.09 | 3.70 | 9.30 |
| | Titan dioxide | 0.62 | 0.35 | 0.62 | 0.35 | 0.62 | 1.55 |
| Total: | | 100.00 | 56.46 | 100.00 | 56.46 | 100.00 | 251.24 |

**Table 27 Extended release layer**

| | | Variant 009 | | Variant 006 | | Variant B | |
|---|---|---|---|---|---|---|---|
| Processing step | Ingredients | % | mg/unit | % | mg/unit | % | mg/unit |
| Extended release layer | Protective layered pellets | 83.33 | 56.46 | 83.33 | 56.46 | 83.33 | 251.24 |
| | Ethyl cellulose | 8.77 | 5.94 | 8.77 | 5.94 | 8.55 | 25.77 |
| | Hydroxy propyl cellulose | 6.14 | 4.16 | 6.14 | 4.16 | 5.98 | 18.04 |
| | Aerosil 200 | 0.88 | 0.59 | 0.88 | 0.59 | 1.28 | 3.87 |
| | Triethyl citrate | 0.88 | 0.59 | 0.88 | 0.59 | 0.85 | 2.58 |
| Total: | | 100.00 | 67.76 | 100.00 | 67.76 | 100.00 | 301.49 |

### Example 19

### Extended release pellets with a high drug load (50%) manufactured with different starter cores

This example describes the impact on drug release due to the used starter cores. In Variant A cellulose containing starter cores (Cellets 100) were used with a particle size between 100 to 200 µm. In Variant 006 sugar spheres (suglets) were used as starter core with a particle size between 250 to 355 µm. The drug release was influenced by the surface area of the starter core and the solubility of the starter core.

The drug substance containing layer was sprayed on the starter cores, followed by a protective layer and an extended release layer using a pore former ratio of 100:70.

### Preparation Drug layer:

Antioxidant butyl hydroxy toluol (2% based on DS) was dissolved in ethanol. The matrix forming polymer HPMC (type 2910, 3 cP, 1:1 based on the DS) and talc was dispersed in ethanol. A small fraction of water (6%) of total amount of solvents was added to the dispersion. During continuous stirring the dispersion was equilibrated until the swollen polymer particles were disintegrated. Finally, the drug substance was added and dispersed in the coating dispersion prior to starting the layering onto the starter cores, preheated and fluidized in a fluid bed processor. The concentration of drug substance was 45% (Variant A) 46% (Variant 006) based on the layer thickness. The spraying occurred at a controlled product bed temperature in the range between 34° and 38°C using a tangential spray process. The obtained multiparticulates were dried in the fluid bed at temperatures up to 55°C.

### Preparation Protective layer:

As described in example 18.

### Preparation Extended release layer:

The extended release polymer ethyl cellulose and the pore former hydroxy propyl cellulose were added and dissolved in ethanol. Afterwards the plasticizer triethylcitrate and anticaking agent Aerosil 200 were added dissolved and dispersed in Ethanol with a final concentration of 8% in the solvent.

The spraying was conducted at a controlled product bed temperature in the range between 34° and 38°C using a bottom spray process until a polymer weight gain of 20% (Variant 006) and 25% (Variant A) was received.

The fill weight was adjusted to amount equivalent to 5 mg and 20mg everolimus fitting in HPMC capsules size 1.

**Table 28 Drug layer**

| | | Variant A | | Variant 006 | |
|---|---|---|---|---|---|
| Pro-cessing step | Ingredients | % | mg/unit | % | mg/unit |
| Drug layer | Sugar spheres 250-355 µm | - | - | 76.92 | 36.20 |
| | Cellets 100 100-200µm | 76.92 | 148.00 | - | - |
| | Everolimus | 10.40 | 20.00 | 10.63 | 5.00 |
| | BHT | 0.21 | 0.40 | 0.23 | 0.11 |
| | Hypromellose 2910 3 cP | 10.40 | 20.00 | 10.63 | 5.00 |
| | Talc | 2.08 | 4.00 | 1.59 | 0.75 |
| Total: | | 100.00 | 192.40 | 100.00 | 47.05 |

**Table 29 Protective layer**

| | | Variant A | | Variant 006 | |
|---|---|---|---|---|---|
| Pro-cessing step | Ingredients | % | mg/unit | % | mg/unit |
| protective layer | Drug layered pellets | 76.92 | 192.40 | 83.33 | 47.05 |
| | Hypromellose 2910 3 cP | 17.09 | 42.76 | 12.35 | 6.97 |
| | Talc | 5.13 | 12.83 | 3.70 | 2.09 |
| | Titan dioxide | 0.85 | 2.14 | 0.62 | 0.35 |
| Total: | | 100.00 | 250.12 | 100.00 | 56.46 |

**Table 30 Extended release layer**

| | | Variant A | | Variant 006 | |
|---|---|---|---|---|---|
| Pro-cessing step | Ingredients | % | mg/unit | % | mg/unit |
| Extended release layer | Protective layered pellets | 80.00 | 250.12 | 83.33 | 56.46 |
| | Ethyl cellulose | 10.26 | 32.08 | 8.77 | 5.94 |
| | Hydroxy propyl cellulose | 7.18 | 22.44 | 6.14 | 4.16 |
| | Aerosil 200 | 1.03 | 3.21 | 0.88 | 0.59 |
| | Triethyl citrate | 1.54 | 4.80 | 0.88 | 0.59 |
| Total: | | 100.00 | 312.66 | 100.00 | 67.76 |

### Example 20 New variants without SDS

### Extended release pellets with a dose of 5mg everolimus with high drug load (50%)

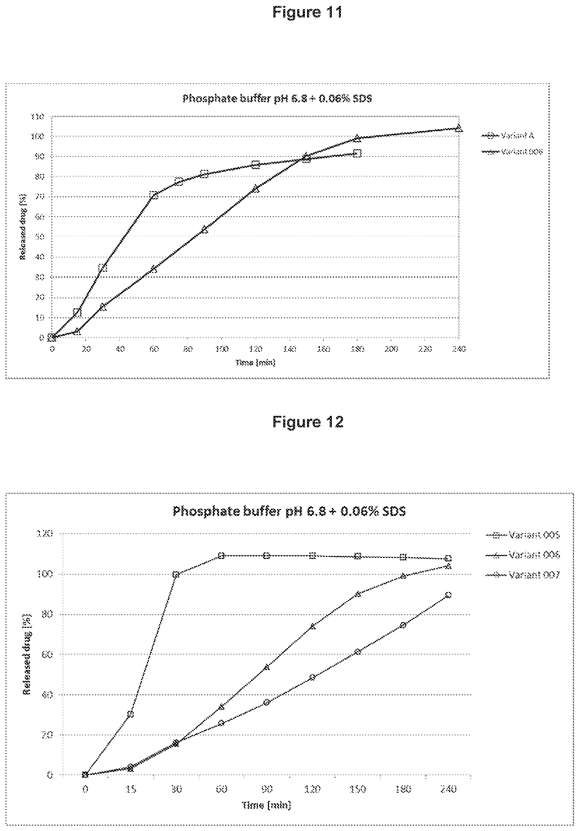

This example describes how the drug release rate (see Figure 12) can be modulated by spraying an extended release layer with a composition comprising a water insoluble polymer (ethyl cellulose) and a pore former (hydroxy propyl cellulose) in different ratios (100:70, 100:55).

The drug substance containing layer was sprayed on the starter cores, followed by a protective layer and finally an extended release layer applied on Variant 006 and 007. Variant 005 contained only the drug layer and the protective layer and represents the immediate release variant.

### Preparation Drug layer:

Antioxidant butyl hydroxy toluol (2% based on DS) was dissolved in ethanol. The matrix forming polymer HPMC (type 2910, 3 cP, 1:1 based on the DS) and talc was dispersed in ethanol. A small fraction of water (6%) of total amount of solvents was added to the dispersion. During continuous stirring the dispersion was equilibrated until the swollen polymer particles were disintegrated. Finally, the drug substance was added and dispersed in the coating dispersion prior to starting the layering onto the starter cores, preheated and fluidized in a fluid bed processor. The concentration of drug substance was about 46% based on the layer thickness. The spraying occurred at a controlled product bed temperature in the range between 34° and 38°C using a tangential spray process. The obtained multiparticulates were dried in the fluid bed at temperatures up to 55°C.

### Preparation Protective layer:

As described in example 18

### Preparation Extended release layer:

The extended release polymer ethyl cellulose and the pore former hydroxy propyl cellulose were added and dissolved in ethanol. Afterwards the plasticizer triethylcitrate and anticaking agent Aerosil 200 were added dissolved and dispersed in Ethanol with a final concentration of 8% in the solvent.

The spraying were conducted at a controlled product bed temperature in the range between 34° and 38°C using a bottom spray process until a polymer weight gain of 20% was received. The fill weight was adjusted to amount equivalent to 5 mg everolimus fitting in HPMC capsules size 1.

**Table 31 Drug layer**

| | | Variant 005, 006, 007 | |
|---|---|---|---|
| Pro-cessing step | Ingredients | % | mg/unit |
| Drug layer | Sugar spheres 250-355 µm | 76.92 | 36.20 |
| | Everolimus | 10.63 | 5.00 |
| | BHT | 0.23 | 0.11 |
| | Hypromellose 2910 3 cP | 10.63 | 5.00 |
| | Talc | 1.59 | 0.75 |
| Total: | | 100.00 | 47.05 |

**Table 32 Protective layer**

| | | Variant 005, 006, 007 | |
|---|---|---|---|
| Pro-cessing step | Ingredients | % | mg/unit |
| protective layer | Drug layered pellets | 83.33 | 47.05 |
| | Hypromellose 2910 3 cP | 12.35 | 6.97 |
| | Talc | 3.70 | 2.09 |
| | Titan dioxide | 0.62 | 0.35 |
| Total: | | 100.00 | 56.46 |

**Table 33 Extended release layer**

| | | Variant 005 | | Variant 006 | | Variant 007 | |
|---|---|---|---|---|---|---|---|
| Processing step | Ingredients | % | mg/unit | % | mg/unit | % | mg/unit |
| Extended release layer | Protective layered pellets | Not performed | | 83.33 | 56.46 | 83.33 | 56.46 |
| | Ethyl cellulose | | | 8.77 | 5.94 | 9.52 | 6.45 |
| | Hydroxy propyl cellulose | | | 6.14 | 4.16 | 5.24 | 3.55 |
| | Aerosil 200 | | | 0.88 | 0.59 | 0.95 | 0.65 |
| | Triethyl citrate | | | 0.88 | 0.59 | 0.95 | 0.65 |
| Total: | | - | - | 100.00 | 67.76 | 100.00 | 67.76 |

### Example 21 New variants without SDS

### Extended release pellets with a dose of 5mg everolimus with high drug load (50%)

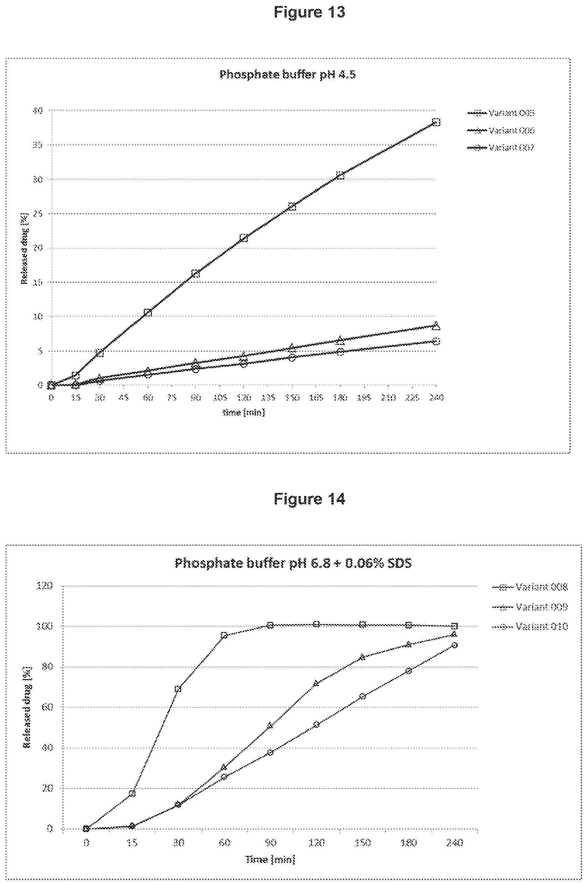

The dissolution of the variants from example 20 was tested in phosphate buffer pH 4.5 to evaluate the influence of the extended release layer on drug release (see Figure 13). Variant 005 represents the variant without extended release coating on top.

The preparation of the drug layer, protective layer and extended release layer was similar as described in Table 31, Table 32 and Table 33.

### Example 22

### Extended release pellets with a dose of 5mg everolimus with high drug load (50%) and additional layer containing surfactant

This example describes how the release rate of an extended release formulation can be modulated by spraying an additional layer containing the surfactant (for example sodium dodecyl sulphate, SDS) on the inert starter core, followed by a drug substance containing layer, a protection layer and an extended release coating. The additional coating layer comprising a surfactant was located beneath the active substance containing layer. This additional layer avoided a direct contact of the surfactant with the active drug substance as well as stabilized the fragile sugar cores prior to drug layering. The desired release properties were fine-tuned by combined action of the surfactant layer, drug substance containing layer and the top coating with extended release properties. The extended release layer of Variant 009 was sprayed with a coating composition comprising a water insoluble polymer (ethyl cellulose) and a pore former (hydroxy propyl cellulose) in a ratio of 100:70 and Variant 010 with the same in a ratio of 100:55. The release profile of this example's formulation is depicted in Figure 14.

### Preparation Surfactant layer:

As described in example 18

### Preparation Drug layer:

The antioxidant butyl hydroxy toluol (2% based on drug substance) and the surfactant were dissolved in ethanol. The matrix forming polymer HPMC (type 2910, 3 cP) and talc were dispersed in the previous ethanol based solution. A small fraction of water (6%) of total amount of solvents was added to the dispersion. During continuous stirring the dispersion was equilibrated until the swollen polymer particles were disintegrated. Finally, the drug substance was added and dispersed in the coating dispersion prior to starting the layering onto the starter cores (sugar spheres or surfactant layered pellets), preheated and fluidized in a fluid bed processor. The concentration of drug substance was 46% based on the drug layer thickness. The spraying occurred at a controlled product bed temperature in the range between 34° and 38°C using a tangential spray process. The obtained multiparticulates were dried in the fluid bed at temperatures up to 55°C.

### Preparation Protective layer:

The preparation of the protective layer was performed as described in example 18.

### Preparation Extended release layer:

The extended release polymer ethyl cellulose and the pore former hydroxy propyl cellulose were added and dissolved in ethanol. Afterwards the plasticizer triethylcitrate and anticaking agent Aerosil 200 were added dissolved and dispersed in Ethanol with a final concentration of 8% in the solvent.

The spraying was conducted at a controlled product bed temperature in the range between 34° and 38°C using a bottom spray process until a polymer weight gain of 20% was received. The fill weight was adjusted to amount equivalent to 5 mg everolismus fitting in HPMC capsules size 1.

**Table 34 Surfactant layer**

| | | Variant 008, 009, 010 | |
|---|---|---|---|
| Pro-cessing step | Ingredients | % | mg/unit |
| Surfactant containing layer | Sugar spheres 250-355 µm | 83.33 | 30.16 |
| | Hypromellose 2910 3 cP | 10.65 | 3.86 |
| | SDS | 2.29 | 0.83 |
| | Talc | 3.20 | 1.16 |
| | Titan dioxide | 0.53 | 0.19 |
| Total: | | 100.00 | 36.20 |

**Table 35 Drug layer**

| | | Variant 008, 009, 010 | |
|---|---|---|---|
| Pro-cessing step | Ingredients | % | mg/unit |
| Drug layering | Surfactant layered pellets | 76.92 | 36.20 |
| | Everolimus | 10.63 | 5.00 |
| | BHT | 0.23 | 0.11 |
| | Hypromellose 2910 3 cP | 10.63 | 5.00 |
| | Talc | 1.59 | 0.75 |
| Total: | | 100.00 | 47.05 |

**Table 36 Protective layer**

| | | Variant 008, 009, 010 | |
|---|---|---|---|
| Pro-cessing step | Ingredients | % | mg/unit |
| protective layer | Drug layered pellets | 83.33 | 47.05 |
| | Hypromellose 2910 3 cP | 12.35 | 6.97 |
| | Talc | 3.70 | 2.09 |
| | Titan dioxide | 0.62 | 0.35 |
| Total: | | 100.00 | 56.46 |

**Table 37 Extended release layer**

| | | Variant 008 | | Variant 009 | | Variant 010 | |
|---|---|---|---|---|---|---|---|
| Processing | Ingredients | % | mg/unit | % | mg/unit | % | mg/unit |
| Extended release layer | Protective layered pellets | Not performed | | 83.33 | 56.46 | 83.33 | 56.46 |
| | Ethyl cellulose | | | 8.77 | 5.94 | 9.52 | 6.45 |
| | Hydroxy propyl cellulose | | | 6.14 | 4.16 | 5.24 | 3.55 |
| | Aerosil 200 | | | 0.88 | 0.59 | 0.95 | 0.65 |
| | Triethyl citrate | | | 0.88 | 0.59 | 0.95 | 0.65 |
| Total: | | - | - | 100.00 | 67.76 | 100.00 | 67.76 |

### Example 23

### Extended release pellets with a dose of 5mg everolimus with high drug load (50%) and additional layer containing surfactant

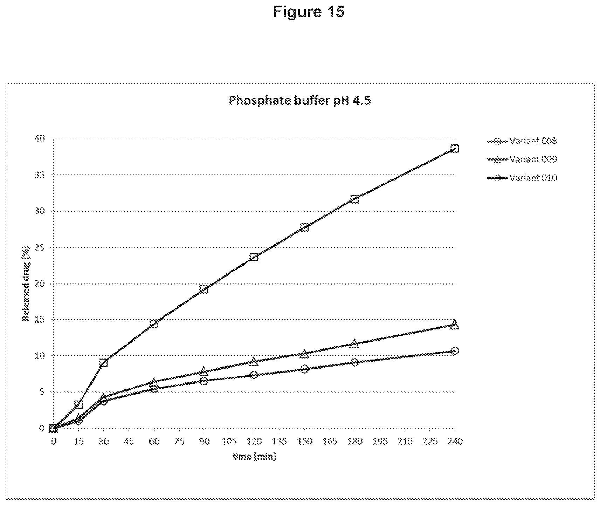

The dissolution of the variants from example 22 was tested in phosphate buffer pH 4.5 to evaluate the influence of the extended release layer on drug release and the increased wettability due to the addition of surfactant. Variant 008 represents the variant without extended release coating on top. The example is illustrated in Figure 15.

The preparation of the surfactant layer, drug layer, protective layer and extended release layer was similar as described in Table 34, Table 35, Table 36 and Table 37.

### Example 24

### Effect of a surfactant layer and a dissolution media on drug release

The release was measured in a dissolution assay with a dissolution vessel filled with 900 mL phosphate buffer pH 4.5 at 37°C by following the paddle method at 75 rpm according to USP <711>, and Ph.Eur. 2.9.3. respectively. The dissolution media proved to be very discriminative and was able to detect differences in dissolution of pellets containing surfactant (Table 38). Composition variants 005, 006, 007, 008, 009 and 010 as described above were used in the dissolution method.

**Table 38**

| **Time** | **pH 4.5** | | | | | |
|---|---|---|---|---|---|---|
| | **without surfactant** | | | **with surfactant** | | |
| | **SC** | **SR**_**70% HPC** | **SR**_**55% HPC** | **SC** | **SR**_**70**% **HPC** | **SR**_**55% HPC** |
| | **005** | **006** | **007** | **008** | **009** | **010** |
| 0 | 0.00 | 0.00 | 0.00 | 0.00 | 0.00 | 0.00 |
| 15 | 1.46 | 0.13 | 0.00 | 3.26 | 1.39 | 1.02 |
| 30 | 4.73 | 1.05 | 0.62 | 9.02 | 4.26 | 3.74 |
| 60 | 10.58 | 2.10 | 1.55 | 14.42 | 6.41 | 5.44 |
| 90 | 16.28 | 3.26 | 2.38 | 19.20 | 7.86 | 6.55 |
| 120 | 21.43 | 4.26 | 3.14 | 23.66 | 9.21 | 7.39 |
| 150 | 26.05 | 5.46 | 4.06 | 27.74 | 10.32 | 8.20 |
| 180 | 30.61 | 6.54 | 4.87 | 31.68 | 11.70 | 9.06 |
| 240 | 38.33 | 8.70 | 6.42 | 38.62 | 14.33 | 10.69 |

Similarly, the dissolution was measured in 900 mL phosphate buffer pH 6.8 containing 0.06 wt% sodium dodecyl sulfate at 37°C by following the paddle method at 75 rpm according to USP <711>, and Ph.Eur. 2.9.3., respectively. The results are shown in Table 39.

**Table 39**

| **Time** | **pH 6.8 + 0.06% SDS** | | | | | |
|---|---|---|---|---|---|---|
| | **without surfactant** | | | **with surfactant** | | |
| | **SC** | **SR**_**70% HPC** | **SR**_**55% HPC** | **SC** | **SR**_**70% HPC** | **SR**_**55% HPC** |
| | **005** | **006** | **007** | **008** | **009** | **010** |
| 0 | 0.00 | 0.00 | 0.00 | 0.00 | 0.00 | 0.00 |
| 15 | 30.21 | 3.21 | 3.96 | 17.31 | 1.53 | 1.27 |
| 30 | 99.74 | 15.58 | 16.16 | 69.16 | 11.90 | 11.88 |
| 60 | 109.21 | 34.08 | 25.61 | 95.58 | 30.51 | 25.63 |
| 90 | 109.19 | 53.83 | 36.02 | 100.63 | 50.89 | 37.76 |
| 120 | 109.11 | 74.07 | 48.46 | 101.03 | 71.76 | 51.41 |
| 150 | 108.80 | 90.20 | 61.31 | 100.72 | 84.73 | 65.44 |
| 180 | 108.35 | 99.15 | 74.68 | 100.55 | 90.98 | 78.02 |
| 240 | 107.71 | 104.21 | 89.59 | 100.03 | 96.06 | 90.68 |

### Figure Legends

Figure 1: in-vitro release profiles of sustained release coated pellets 5mg everolimus in phosphate buffer 6.8, comparison between examples, (Δ) protection layer coated immediate release (example 1/table1), (□) sustained release coated (example 5/table 6), (◊) sustained release coated (example 5/table 8), (○) sustained release coated (example 5/table 9).
Figure 2: in-vitro release profiles of sustained release coated pellets 5mg everolimus in phosphate buffer 6.8, comparison between examples, (Δ) protection layer coated immediate release (example 1/table1), (□) sustained release coated with EC and HPMC as pore former(example 6/ table 10), (◊) sustained release coated with Eudragit RS/RL 3:7(example 3/ table 4).
Figure 3: in-vitro release profiles of sustained release coated minitablets in phosphate buffer 6.8, (□) coated with EC and HPC as pore former (example 8/ table 14).
Figure 4: in-vitro release profiles of sustained release coated pellets coated with EC and HPC as pore former in phosphate buffer 6.8, comparison between examples, (□) 10mg tablet formulation with pellets (example 10 table 16), (Δ) 20mg pellets (example 9/ table 15).
Figure 5: in-vitro release profiles of sustained release coated pellets coated with EC and HPC as pore former in phosphate buffer 6.8, comparison between examples, (◊) 5mg formulation (example 5/ table 8), (Δ) 20mg formulation (example 9/ table 15).
Figure 6: simulation of plasma concentration curve with multiple doses of 10mg everolimus in fed and fasted state comparing 3 different formulations:
   IR: conventional, immediate release, fast disintegrating tablet (top line)
   SR 6h: sustained release pellets in a HPMC capsule size 0, 5mg everolimus per capsule; approximately 90% everolimus released in 3h, example 5 / table 6 (two bottom lines)
   SR 3h: sustained release pellets in a HPMC capsule size 0, 5mg everolimus per capsule; approx. 90% everolimus released in 3h, example 5 / table 7 (remaining two middle lines)
Figure 7: Simulated concentration-time profiles after daily administration of 10 mg FMI after a light fat meal, 10 mg SR-fast variant under fasting conditions, and a combination of 1 mg immediate release in a combination with 10 mg sustained release, i.e. fast variant (IR+SR) under fasting condition at state-state
Figure 8: Dissolution results obtained with a pure amorphous everolimus.
Figure 9: Dissolution of solid dispersion powder containing 20 wt% and 80 wt% Everolimus content.
Figure 10: in-vitro release profiles of extended release coated pellets coated with EC and HPC as pore former (100:70) in phosphate buffer pH 4.5, (○) the surfactant is located in the surfactant layer, (□) the surfactant is located in the drug layer, (Δ) without surfactant (see example 18).
Figure 11: in-vitro release profiles of extended release coated pellets coated with EC and HPC as pore former (100:70) in phosphate buffer pH 6.5 with 0.06% SDS, (□) pellets 100 were used as starter cores, (Δ) without surfactant (see example 19).
Figure 12: in-vitro release profiles of protective layered pellets and extended release coated pellets with EC as sustained release polymer and HPC as pore former; different pore former ratios were applied (100:70, 100:55); as medium phosphate buffer pH 6.5 with 0.06% SDS is used, (□) without extended release layer, (Δ) with a pore former ratio of 100:70 (○) with a pore former ratio of 100:55; (see example 19).
Figure 13: in-vitro release profiles of protective layered pellets and extended release coated pellets with EC as extended release polymer and HPC as pore former; different pore former ratios were applied (100:70, 100:55); as medium phosphate buffer pH 4.5 is used, (□) without extended release layer, (Δ) with a pore former ratio of 100:70 (○) with a pore former ratio of 100:55; (see example 20).
Figure 14: in-vitro release profiles of protective layered and extended release pellets containing an additional surfactant layer beneath the drug layer to improve wettability of drug substance; the extended release layer was sprayed with EC as extended release polymer and HPC as pore former; different pore former ratios were applied (100:70, 100:55); as medium phosphate buffer pH 6.5 with 0.06% SDS is used, (□) without extended release layer, (Δ) with a pore former ratio of 100:70 (○) with a pore former ratio of 100:55; (see example 21).
Figure 15: in-vitro release profiles of protective layered and extended release pellets containing an additional surfactant layer beneath the drug layer to improve wettability of drug substance; the extended release layer was sprayed with EC as extended release polymer and HPC as pore former; different pore former ratios were applied (100:70, 100:55); as medium phosphate buffer pH 4.5, (□) without extended release layer, (Δ) with a pore former ratio of 100:70 (○) with a pore former ratio of 100:55; (see example 22).

## Claims

1. A pharmaceutical formulation comprising a first part and a second part, wherein the first part comprises a layer with, based on the weight of the layer, more than 40 wt % of 40-O-(2-hydroxy)ethyl-rapamycin, and the second part releases more than 85 wt % of 40-O-(2-hydroxy)ethyl-rapamycin of the second part in less than 60 minutes.

2. A pharmaceutical formulation according to claim 1, wherein the first part comprises a layer with, based on the weight of the layer, between 50 to 80 wt % of 40-O-(2-hydroxy)ethyl-rapamycin.

3. A pharmaceutical formulation according to claim 1 or claim 2, wherein the first part and/or the second part is in a form of a minitablet, pellet, microparticle, microcapsule, granule, bead, tablet, a coating layer of a coated minitablet, pellet, microparticle, microcapsule, granule, bead, tablet, or a layer of a double or multilayer tablet.

4. A pharmaceutical formulation according to any one of the previous claims, wherein the first part is in a form of a coating and the second part is in a form of a coating.

5. A pharmaceutical formulation according to any one of the previous claims, wherein the formulation further comprises a surfactant.

6. A pharmaceutical formulation according to claim 5, wherein the surfactant is in a coating, wherein the coating with the surfactant is enclosed at least by the layer with, based on the weight of the layer, more than 40 wt % of 40-O-(2-hydroxy)ethyl-rapamycin.

7. A pharmaceutical formulation comprising 40-O-(2-hydroxy)ethyl-rapamycin in a first layer and a surfactant in a second layer, wherein the second layer is beneath the first layer.

8. A pharmaceutical formulation comprising 40-O-(2-hydroxy)ethyl-rapamycin in a first layer and a surfactant in a second layer according to claim 7, wherein the first and the second layer are coatings.

9. A pharmaceutical formulation comprising 40-O-(2-hydroxy)ethyl-rapamycin in a first layer and a surfactant in a second layer according to claim 7 or 8, wherein the second layer with the surfactant is enclosed at least by the first layer.

10. A pharmaceutical formulation comprising 40-O-(2-hydroxy)ethyl-rapamycin in a first layer and a surfactant in a second layer according to any one of claims 7 to 9, wherein 40-O-(2-hydroxy)ethyl-rapamycin in the first layer is in a solid dispersion and the solid dispersion comprises 18 to 50 wt % of 40-O-(2-hydroxy)ethyl-rapamycin.

11. A pharmaceutical formulation according to any one of claims 5 to 10, wherein the surfactant is polyoxyethylene-polyoxypropylene co-polymer or block co-polymer, polyoxyethylene sorbitan fatty acid ester, polyoxyethylene fatty acid ester, poly-oxyethylene alkyl ether, sodium alkyl sulfate or sulfonate, sodium alkyl aryl sulfonate, water soluble tocopheryl polyethylene glycol succinic acid ester, polyglycerol fatty acid ester, alkylene polyol ether or ester, polyethylene glycol glyceryl fatty acid ester, sterol, transesterified and polyoxyethylated caprylic-capric acid glyceride, sugar fatty acid ester, PEG sterol ether, phospholipids, salts of a fatty acid, fatty acid sulfate or sulfonate, salt of fatty acid, fatty acid sulfate or sulfonate, medium or long-chain alkyl ammonium salt, bile acid or salt thereof, glycolic acid or a salt, polyoxyethylene mono ester of a saturated C10 to C22 fatty acid, or a combination thereof.

12. A pharmaceutical formulation according to any one of claims 5 to 11, wherein the surfactant is polyoxyethylene-polyoxypropylene co-polymer or block co-polymer or a water soluble tocopheryl polyethylene glycol succinic acid ester.

13. A pharmaceutical formulation according to any one of claims 1 to 12, wherein the formulation comprises crospovidone.

14. A pharmaceutical formulation according to any one of claims 5 to 13, wherein the surfactant is vitamin E polyethylene glycol 1000 succinate, poloxamer 188, sodium lauryl sulfate, or combinations thereof.

15. A pharmaceutical formulation according to any one of claims 1 to 14, further comprising a desiccant.

## Patentansprüche

1. Pharmazeutische Formulierung, umfassend einen ersten Teil und einen zweiten Teil, wobei der erste Teil eine Schicht mit, bezogen auf das Gewicht der Schicht, mehr als 40 Gew.-% 40-O-(2-Hydroxy)ethylrapamycin umfasst und der zweite Teil in weniger als 60 Minuten mehr als 85 Gew.-% von 40-O-(2-Hydroxy)ethylrapamycin des zweiten Teils freisetzt.

2. Pharmazeutische Formulierung nach Anspruch 1, wobei der erste Teil eine Schicht mit, bezogen auf das Gewicht der Schicht, zwischen 50 und 80 Gew.-% 40-O-(2-Hydroxy)ethylrapamycin umfasst.

3. Pharmazeutische Formulierung nach Anspruch 1 oder Anspruch 2, wobei der erste Teil und/oder der zweite Teil in Form einer Minitablette, eines Pellets, eines Mikropartikels, einer Mikrokapsel, eines Körnchens, einer Perle, einer Tablette, einer Beschichtungsschicht einer beschichteten Minitablette, eines beschichteten Pellets, eines beschichteten Mikropartikels, einer beschichteten Mikrokapsel, eines beschichteten Körnchens, einer beschichteten Perle, einer beschichteten Tablette oder einer Schicht einer Doppel- oder Multischichttablette vorliegt.

4. Pharmazeutische Formulierung nach einem der vorhergehenden Ansprüche, wobei der erste Teil in Form einer Beschichtung vorliegt und der zweite Teil in Form einer Beschichtung vorliegt.

5. Pharmazeutische Formulierung nach einem der vorhergehenden Ansprüche, wobei die Formulierung ferner ein Tensid umfasst.

6. Pharmazeutische Formulierung nach Anspruch 5, wobei sich das Tensid in einer Beschichtung befindet, wobei die Beschichtung mit dem Tensid mindestens von der Schicht mit, bezogen auf das Gewicht der Schicht, mehr als 40 Gew.-% 40-O-(2-Hydroxy)ethylrapamycin umschlossen ist.

7. Pharmazeutische Formulierung, umfassend 40-O-(2-Hydroxy)ethylrapamycin in einer ersten Schicht und ein Tensid in einer zweiten Schicht, wobei sich die zweite Schicht unter der ersten Schicht befindet.

8. Pharmazeutische Formulierung, umfassend 40-O-(2-Hydroxy)ethylrapamycin in einer ersten Schicht und ein Tensid in einer zweiten Schicht nach Anspruch 7, wobei es sich bei der ersten und der zweiten Schicht um Beschichtungen handelt.

9. Pharmazeutische Formulierung, umfassend 40-O-(2-Hydroxy)ethylrapamycin in einer ersten Schicht und ein Tensid in einer zweiten Schicht nach Anspruch 7 oder 8, wobei die zweite Schicht mit dem Tensid mindestens von der ersten Schicht umschlossen ist.

10. Pharmazeutische Formulierung, umfassend 40-O-(2-Hydroxy)ethylrapamycin in einer ersten Schicht und ein Tensid in einer zweiten Schicht nach einem der Ansprüche 7 bis 9, wobei 40-O-(2-Hydroxy)ethylrapamycin in der ersten Schicht in einer festen Dispersion vorliegt und die feste Dispersion 18 bis 50 Gew.-% 40-O-(2-Hydroxy)ethylrapamycin umfasst.

11. Pharmazeutische Formulierung nach einem der Ansprüche 5 bis 10, wobei es sich bei dem Tensid um Polyoxyethylen-Polyoxypropylen-Copolymer oder -Blockcopolymer, Polyoxyethylensorbitanfettsäureester, Polyoxyethylenfettsäureester, Polyoxyethylenalkylether, Natriumalkylsulfat oder -sulfonat, Natriumalkylarylsulfonat, wasserlöslichen Tocopherylpolyethylenglykolbernsteinsäureester, Polyglycerinfettsäureester, Alkylenpolyolether oder -ester, Polyethylenglykolglycerylfettsäureester, Styrol, umgeesteres und polyoxyethyliertes Capryl-Caprinsäuretriglycerid, Zuckerfettsäureester, PEG-Sterolether, Phospholipide, Salze einer Fettsäure oder eines Fettsäuresulfats oder -sulfonats, Salz von Fettsäure, Fettsäuresulfat oder -sulfonat, mittel- oder langkettiges Alkylammoniumsalz, Gallensäure oder ein Salz davon, Glykolsäure oder ein Salz, Polyoxyethylenmonoester einer gesättigten C10- bis C22-Fettsäure oder eine Kombination davon handelt.

12. Pharmazeutische Formulierung nach einem der Ansprüche 5 bis 11, wobei es sich bei dem Tensid um Polyoxyethylen-Polyoxypropylen-Copolymer oder -Blockcopolymer oder einen wasserlöslichen Tocopherylpolyethylenglykolbernsteinsäureester handelt.

13. Pharmazeutische Formulierung nach einem der Ansprüche 1 bis 12, wobei die Formulierung Crospovidon umfasst.

14. Pharmazeutische Formulierung nach einem der Ansprüche 5 bis 13, wobei es sich bei dem Tensid um Vitamin-E-polyethylenglykol-1000-succinat, Poloxamer 188, Natriumlaurylsulfat oder Kombinationen davon handelt.

15. Pharmazeutische Formulierung nach einem der Ansprüche 1 bis 14, ferner umfassend ein Trockenmittel.

## Revendications

1. Formule pharmaceutique comprenant une première partie et une deuxième partie, où la première partie comprend une couche comportant, par rapport à la masse de la couche, plus de 40 % en masse de 40-O-(2-hydroxy)éthyl-rapamycine, et la deuxième partie libère plus de 85 % en masse de 40-O-(2-hydroxy)éthylrapamycine de la deuxième partie en moins de 60 minutes.

2. Formule pharmaceutique selon la revendication 1, où la première partie comprend une couche comportant, par rapport à la masse de la couche, entre 50 et 80 % en masse de 40-O-(2-hydroxy)éthylrapamycine.

3. Formule pharmaceutique selon la revendication 1 ou la revendication 2, où la première partie et/ou la deuxième partie se présentent sous la forme d'un minicomprimé, d'une bille, d'une microparticule, d'une microcapsule, d'un granule, d'une perle, d'un comprimé, d'une couche d'enrobage d'un minicomprimé, d'une bille, d'une microparticule, d'une microcapsule, d'un granule, d'une perle, d'un comprimé enrobés, ou d'une couche d'un comprimé bicouche ou multicouche.

4. Formule pharmaceutique selon l'une quelconque des revendications précédentes, où la première partie se présente sous la forme d'un enrobage et la deuxième partie se présente sous la forme d'un enrobage.

5. Formule pharmaceutique selon l'une quelconque des revendications précédentes, où la formule comprend en outre un tensioactif.

6. Formule pharmaceutique selon la revendication 5, où le tensioactif est contenu dans un enrobage, où l'enrobage avec le tensioactif est enfermé au moins par la couche comportant, par rapport à la masse de la couche, plus de 40 % en masse de 40-O-(2-hydroxy)éthylrapamycine.

7. Formule pharmaceutique comprenant de la 40-O-(2-hydroxy)éthyl-rapamycine dans une première couche et un tensioactif dans une deuxième couche, où la deuxième couche est située en dessous de la première couche.

8. Formule pharmaceutique comprenant de la 40-O-(2-hydroxy)éthyl-rapamycine dans une première couche et un tensioactif dans une deuxième couche selon la revendication 7, où la première et la deuxième couche sont des enrobages.

9. Formule pharmaceutique comprenant de la 40-O-(2-hydroxy)éthyl-rapamycine dans une première couche et un tensioactif dans une deuxième couche selon la revendication 7 ou 8, où la deuxième couche contenant le tensioactif est enfermée dans au moins la première couche.

10. Formule pharmaceutique comprenant de la 40-O-(2-hydroxy)éthyl-rapamycine dans une première couche et un tensioactif dans une deuxième couche selon l'une quelconque des revendications 7 à 9, où la 40-O-(2-hydroxy)éthyl-rapamycine dans la première couche se présente sous forme d'une dispersion solide et la dispersion solide comprend 18 à 50 % en masse de 40-O-(2-hydroxy)éthyl-rapamycine.

11. Formule pharmaceutique selon l'une quelconque des revendications 5 à 10, où le tensioactif est un copolymère ou un copolymère bloc polyoxyéthylène-polyoxypropylène, un ester d'acide gras de polyoxyéthylène sorbitane, un ester d'acide gras de polyoxyéthylène, un poly-oxyéthylène alkyléther, un alkylsulfate ou sulfonate de sodium, un alkylarylsulfonate de sodium, un ester d'acide succinique de tocophérylpolyéthylène glycol hydrosoluble, un ester d'acide gras de polyglycérol, un ester ou éther d'alkylène polyol, un ester d'acide gras de polyéthylène glycol glycéryle, un stérol, un glycéride d'acide caprylique-caprique transestérifié et polyoxyéthylé, un ester d'acide gras de sucre, un éther de PEG et de stérol, des phospholipides, des sels d'un acide gras, un sulfate ou sulfonate d'acide gras, un sel d'acide gras, de sulfate ou de sulfonate d'acide gras, un sel d'alkylammonium à chaîne moyenne ou longue, l'acide biliaire ou l'un de ses sels, l'acide glycolique ou l'un de ses sels, un monoester de polyoxyéthylène d'un acide gras en C10 à C22 saturé, ou l'une de leurs combinaisons.

12. Formule pharmaceutique selon l'une quelconque des revendications 5 à 11, où le tensioactif est un copolymère ou copolymère bloc de polyoxyéthylène-polyoxypropylène ou un ester d'acide succinique de tocophérylpolyéthylène glycol hydrosoluble.

13. Formule pharmaceutique selon l'une quelconque des revendications 1 à 12, où la formule comprend de la crospovidone.

14. Formule pharmaceutique selon l'une quelconque des revendications 5 à 13, où le tensioactif est le succinate de vitamine E polyéthylène glycol 1000, le poloxamère 188, le laurylsulfate de sodium ou leurs combinaisons.

15. Formule pharmaceutique selon l'une quelconque des revendications 1 à 14, comprenant en outre un agent déshydratant.
